# EUROPEAN PATENT SPECIFICATION

(11) **EP 0 291 787 B1**
(45) Date of publication and mention of the grant of the patent: **16.03.1994**
(21) Application number: 88107312.6
(22) Date of filing: 06.05.1988
(51) Int. Cl.: C07F 7/08, A61K 31/695

(54) **Novel substituted silyl alkylene amines**
Substituierte Silylalkylenamine
Silyle alkylène amines substituées

(30) Priority: 08.05.1987 US 47847
(43) Date of publication of application: 23.11.1988
(73) Proprietor: MERRELL DOW PHARMACEUTICALS INC., Cincinnati Ohio 45215-6300 (US)
(72) Inventor: Schirlin, Daniel, F-67800 Bischheim (FR); Collard, Jean-Noel, F-67800 Bischheim (FR); Danzin, Charles, F-67000 Strasbourg (FR)
(74) Representative: VOSSIUS & PARTNER

(56) References cited:
- BULL. CHEM. SOC. JPN., vol. 58, July 1985, The ChemICAL Society of Japan, Tokyo (JP); O. TSUGE et al., pp. 1991-1999#
- J. ORG. CHEM., vol. 45, no. 4, 1980, American Chemical Society, Washington, DC (US); Y. SATO et al., pp. 613-617#
- J. ORG. CHEM., vol. 50, no. 21, 1985, American Chemical Society, Washington, DC (US); A. PADWA et al., pp. 4006-4014#
- JOURNAL OF GENERAL CHEMISTRY OF THE USSR, vol. 54, no. 9, September 1984, part 1, Plenum Publishing Corp.; E.A. CHERNYSHEV et al., pp. 1812-1815#
- JOURNAL OF ORGANOMETALLIC CHEMISTRY, vol. 248, 1983, Elsevier Sequoia S.A., Lausanne (CH); D.A. BRAVO-ZHIVOTOVSKII et al., pp. 51-60#
- JOURNAL OF THE AMERICAN CHEMICAL SOCIETY, vol. 96, no. 14, 10 July 1974, Washington, DC (US); A.G. BROOK et al., pp. 4692-4693#
- JOURNAL OF ORGANOMETALLIC CHEMISTRY, vol. 153, 1978, Elsevier Sequoia S.A., Lausanne (CH); T. AOYAMA et al., pp. 193-207#
- JOURNAL OF ORGANOMETALLIC CHEMISTRY, vol. 178, 1979, Elsevier Sequoia S.A., Lausanne (CH); R.A. BENKESER et al., pp. 21-28#
- JOURNAL OF GENERAL CHEMISTRY OF THE USSR, vol. 45, no. 2, February, 1975, Plenum Publishing Co., New York, NY (US); K.A. ANDRIANOV et al., pp. 340-345#
- Z. ANORG. ALLG. CHEM., vol. 476, 1981; V.G. ZINGLER etal., pp. 41-54#
- CHEMICAL ABSTRACTS, vol. 72, no. 7, 16 February 1970, Columbus, OH (US); E. LUKEVICS et al., p. 356, no. 31906r#
- CHEMICAL ABSTRACTS, vol. 63, no. 7, 27 September 1965, Columbus, OH (US); T.A. SLADKOVA et al., no. 8395c#
- CHEMICAL ABSTRACTS, vol. 64, no. 12, 06 June 1966, Columbus, OH (US); N.S. NAMETKIN et al., no. 17625fg#
- CHEMICAL ABSTRACTS, vol. 67, no. 15, 09 October 1967, Columbus, OH (US); A.K. PROKOF'EV et al., p. 6945, no. 73657n#
- INORGANIC CHEMISTRY, vol. 4, no. 10, October 1965; N.E.MILLER, pp. 1458-1463#
- CHEMICAL ABSTRACTS, vol. 70, no. 7, 17 February 1969, Columbus, OH (US); E. LUKEVICS et al., p. 330, no. 28982r#
- CHEMICAL ABSTRACTS, vol. 86, no. 1, 03 January 1977, Columbus, OH (US); E. LUKEVICS et al., p. 480, no. 5520e#
- CHEMICAL ABSTRACTS, vol. 90, no. 15, 09 April 1979, Columbus, OH (US); E. LUKEVICS, p. 27, no. 114916s#
- CHEMICAL ABSTRACTS, vol. 76, no. 19, 08 May 1972, Columbus, OH (US); C. BIRAN et al., p. 495, no. 113295n#
- CHEMISCHE BERICHTE, vol. 118, 1985, VCH VerlagsgesmbH, Weinheim (DE); J. VOSS et al., pp. 4806-4820#
- JOURNAL OF ORGANOMETALLIC CHEMISTRY, vol. 113, 1976, Elsevier Sequoia S.A., Lausanne (CH); Y. SATO et al., pp. 115-125#
- CHEMICAL ABSTRACTS, vol. 82, no. 9, 03 March 1975, Columbus, OH (US); E. LUKEVICS et al., p. 584, no. 57805p#
- E. Lukevics, BIOLOGICAL ACTIVITY OF NITROGEN-CONTAINING ORGANOSILICON COMPOUNDS, Nobel Symp. 1977, publ. 1978, vol. 40; pp. 435-445#
- E. Juckner, FORTSCHRITTE DER ARZNEIMITTELFORSCHUNG, vol. 7, 1964; pp. 306-307#
- MEDICAL CHEMISTRY, 3rd ed., part 1, 1979; A. BURGER, p. 78#

## Description

This invention relates to substituted silyl alkylene amines, to the intermediates and processes useful for their preparation, to their pharmacological use as MAO-B inhibitors and to their end-use application in the treatment of Parkinson's Disease.

More specifically this invention relates to substituted silyl alkylene amines of the formula 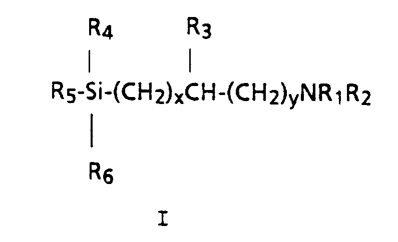
and the pharmaceutically acceptable salts thereof, wherein each of x and y is zero or one, with the proviso that the sum of x and y is less than two,
- R₁: is H or C₁₋₁₀ alkyl,
- R₂: is H, C₁₋₁₀ alkyl, (CH₂)ₘ phenyl with m being 1 to 4,
- R₃: is H or (CH₂)ₙR₃' with n being zero or 1 to 4
- R₄: is C₁₋₁₀ alkyl,
- R₅: is (CH₂)ₘR₅' with m being 1 to 4,
- R₆: is (CH₂)ₚR₆' with p being 1 to 4, with R₃', R₅' and R₆' being H, C₁₋₁₀ alkyl, aryl or X,Y substituted aryl, each of X and Y being C₁₋₁₀ alkyl, C₁₋₁₀ alkoxy, CF₃, halogeno, OH, CN or NO₂, with the proviso that when x and y are zero and R₃ is H then the SiR₄R₅R₆ moiety is other than trimethylsilyl, triethylsilyl, benzyldimethylsilyl, or benzyldiethylsilyl and the further provisos that when x is zero and y is one and SiR₄R₅R₆ is trimethylsilyl then R₃ is other than H, and when y is zero and x is one and SiR₄R₅R₆ is trimethylsilyl then R₃ is other than H, methyl or phenyl, and that

Me₃SiCH(Ph)NH₂, Me₃SiCH(o-MePh)NMe₂, Me₃SiCH(Me)NHBz, Et₃Si(CH₂)₃NEt₂, Et₃Si(CH₂)₃N(n-Pr)₂, Et₃Si(CH₂)₃N(n-Bu)₂, Et₃SiCH(Ph)NEt₂, Me₂(m-ClPhCH₂)SiCH₂NHMe, Me₂(m-ClPhCH₂)SiCH₂NHEt, Me₂(m-ClPhCH₂)SiCH₂NHBz, ME₃SiCH(Ph)NMe₂, Me₃SiCH(p-ClPh)NMe₂, Me₃SiCH(p-MePh)NMe₂, Me₃SiCH(p-MeOPh)NMe₂, Me₃SiCH(Me)NHPh, Me₂EtSiCH₂NH₂, Bz₂MeSiCH₂NEt₂, Bz₂MeSiCH₂NMe₂, EtMe₂SiCH₂NH(n-Bu), (n-Pr)Me₂SiCH₂NH(n-Bu), (n-Bu)Me₂SiCH₂NH(n-Bu), MeEt₂SiCH₂NH(n-Bu), Et₃SiCH(Me)CH₂NH₂, Et₃Si(CH₂)₂NEt₂, Me₃SiCH(Me)NMe₂, MeEt₂SiCH₂NH₂, Et₂MeSiCH₂NH(n-Bu), (n-Bu)₃Si(CH₂)₂NEt₂, Me₃SiCH(α-naphthyl)NH₂, Me₃SiCH(β-naphthyl)NH₂, Me₃SiCH(Ph)NMe₂, Me₃SiCH(Me)NEt₂, Me₃SiCH(Et)N(n-Pr)₂ are excluded.

As used herein the term C₁₋₁₀ alkyl includes the straight, branched-chain and cyclized manifestations thereof, particulary such moieties as methyl, ethyl, n-butyl, t-butyl, cyclopropyl, n-propyl, pentyl, n-hexyl, n-nonyl, decyl, cyclopentyl and cyclohexyl. The term "aryl" within the definitions of the R₃', R₅', and R₆' groups includes both carbocyclic and heterocyclic moieties of which phenyl, pyridyl, indolyl, indazolyl, furyl and thienyl are of primary interest. Of course, these moieties include all of the position isomers thereof such as, for example, 2-, 3-, or 4-pyridyl, 2- or 3-furyl, 2- or 3-thienyl, 1-, 2- or 3-indolyl, and the 1- and 3-indazolyl, as well as the dihydro and tetrahydro analogs of the furyl and thienyl moieties. Also included within the term "aryl" are such fused carbocyclic moieties as pentalenyl, indenyl, naphthalenyl, azulenyl, heptalenyl, acenaphthylenyl, fluorenyl, phenalenyl, phenanthrenyl, anthracenyl, acephenanthrylenyl, aceanthrylenyl, triphenylenyl, pyrenyl, chrysenyl and naphthacenyl. Also included within the term "aryl" are such other heterocyclic radicals as 2- or 3-benzo[b]thienyl, 2- or 3-naphtho[2,3-b]thienyl, 2- or 3-thianthrenyl, 2H-pyran-3-(or 4- or 5-)yl, 1-isobenzofuranyl, 2H-chromen-3-yl, 2- or 3-xanthenyl, 2- or 3-phenoxathiinyl, 2- or 3-pyrrolyl, 4- or 3-pyrazolyl, 2-pyrazinyl, 2-pyrimidinyl, 3-pyridazinyl, 2-indolizinyl, 1-isoindolyl, 4H-quinolizin-2-yl, 3-isoquinolyl, 2-quinolyl, 1-phthalazinyl, 1,8-naphthyridinyl, 2-quinoxalinyl, 2-quinazolinyl, 3-cinnolinyl, 2-pteridinyl, 4aH-carbazol-2-yl, 2-carbazolyl, β-carbolin-3-yl, 3-phenanthridinyl, 2-acridinyl, 2-perimidinyl, 1-phenazinyl, 3-isothiazolyl, 2-phenothiazinyl, 3-isoxazolyl, 2-phenoxazinyl, 3-isochromanyl, 7-chromanyl, 2-pyrrolidinyl, 2-pyrrolin-3-yl, 2-imidazolidinyl, 2-imidazolin-4-yl, 2-pyrazolidinyl, 3-pyrazolin-3-yl, 2-piperidyl, 2-piperazinyl, 1-indolinyl, 1-isoindolinyl, 3-morpholinyl, benzo[h]isoquinolinyl, and benzo[b]furanyl, including the position isomers thereof except that the heterocyclic moieties cannot be attached directly through their nitrogen atoms.

Halogen includes all four members of the group with fluoro and chloro being preferred and fluoro being most preferred. In those instances wherein an aryl moiety bears an X and/or Y substituent, the substituents may be located at any of the normally acceptable positions. In the special instance wherein aryl is phenyl, particularly wherein the substituent is fluoro or chloro (or these in combination with other radicals, e.g., methyl) then the scope of this invention includes the mono-, di-, tri-, tetra- and penta-substituted phenyl moieties with the 4-fluoro, 3-fluoro, 2-fluoro, 2,3-difluoro, 2,4-difluoro, 3,4-difluoro, 3,4,5-trifluoro, 2,4,6-trifluoro, 2,4,5-trifluoro, 2,3,4-trifluoro, 2,5-difluoro, 2,6-difluoro, 3,5-difluoro, 2,3,5-trifluoro, 2,3,6-trifluoro, 2,3,4,5-tetrafluoro, 2,3,4,6-tetrafluoro, 2,3,5,6-tetrafluoro, and the 2,3,4,5,6-pentafluorophenyl substituted moieties being preferred.

The pharmaceutically acceptable salts of the compounds of formula I include salts formed with non-toxic organic or inorganic acids such as, for example, from the following acids: hydrochloric, hydrobromic, sulfonic, sulfuric, phosphoric, nitric, maleic, fumaric, benzoic, ascorbic, pamoic, succinic, methanesulfonic acid, acetic, propionic, tartaric, citric, lactic, malic, mandelic, cinnamic, palmitic, itaconic and benzenesulfonic.

The preparation of the compounds of formula I is effected by a variety of procedures depending primarily upon the specific definition of the x, y, and n designations as well as the definitions for the R₃, R₄, R₅ and R₆ moieties. In any event, the chemical reactions and procedures are analogously known in the art and the selection of a particular route to obtain any particular specific type of compound is governed by principles well known and appreciated by those of ordinary skill in the art.

The following routes of synthesis will serve to teach those of ordinary skill in the art how the compounds of formula I may be prepared.

For the most part the procedures utilize silyl derivatives obtained from tetrachlorosilane (SiCl₄) or trichlorochloromethyl silane (Cl₃SiCH₂Cl) or a tailored modification thereof as starting materials. In essence these silyl derivatives are of the formulae 2 and 3, with R₄, R₅ and R₆ being as defined for formula I or they are in a reaction-protected form of the R₄, R₅, and R₆ substituents. (N.B., For convenience in the following text, formulae 2 and 3 will be described as R₄R₅R₆SiCl and R₄R₅R₆SiCH₂Cl, respectively.) The preparation of the R₄, R₅ and R₆ substituted silanes of formulas 2 and 3 is readily 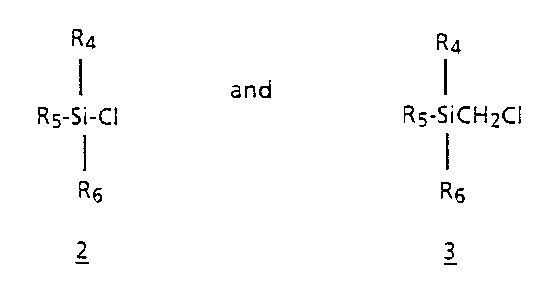
effected by successive alkylations of tetrachloro-silane and trichlorochloromethyl silane using organo-magnesium halide derivatives of the appropriate R₄, R₅ and/or R₆ substituents. For example, SiCl₄ is reacted with R₄Mg halides to produce R₄SiCl₃ compounds which are reacted with R₅Mg halides to produce R₄R₅SiCl₂ compounds which are reacted with R₆Mg halides to produce R₄R₅R₆SiCl compounds. Analogously, R₄Si(Cl₂)CH₂Cl, R₄R₅Si(Cl)CH₂Cl and R₄R₅R₆SiCH₂Cl compounds are prepared by these successive alkylation procedures utilizing Cl₃SiCH₂Cl as a starting reactant.

In general, the method of synthesis in the preparation of any particular compound of formula I will depend upon the nature and definition for the x, y, n and R₃ moieties.

In the instance wherein it is desired to prepare compounds of formula I wherein n, x and y are zero and R₃ is B, the appropriate R₄R₅R₆silyl methyl chloride 3 is subjected to a displacement reaction by treatment with potassium phthalimide or sodium azide to obtain the corresponding phthalimide or azide. Conversion of the phthalimide to the desired amine is by reaction with hydrazine hydrate and conversion of the azide is through chemical reduction to its amine, and subsequent purification of the so-prepared amine may be accomplished via its N-Boc derivative which is converted to the amine by hydrolysis. These reactions are depicted in Reaction Schemes A-1 and A-2. 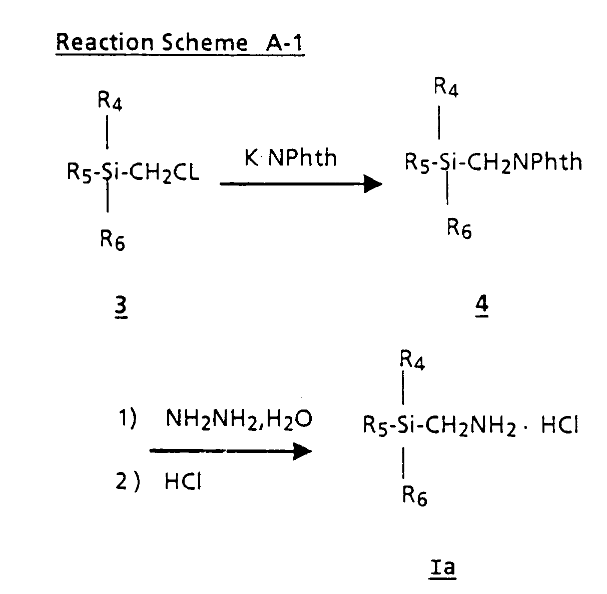
wherein R₄,R₅,R₆ are as defined in formula I and NPhth is a phthalimide moiety.

In effecting the foregoing reaction, the formation of the phthalimide is readily accomplished by standard reaction conditions for the displacement reaction, preferably by heating the reactants in an inert solvent, e.g., dry dimethylformamide at 70°C. The conversion of the phthalimide to its corresponding amine is effected by reaction with hydrazine hydrate in a suitable solvent, preferably ethanol, followed by treatment with aqueous acid, preferably HCl, under reflux conditions. 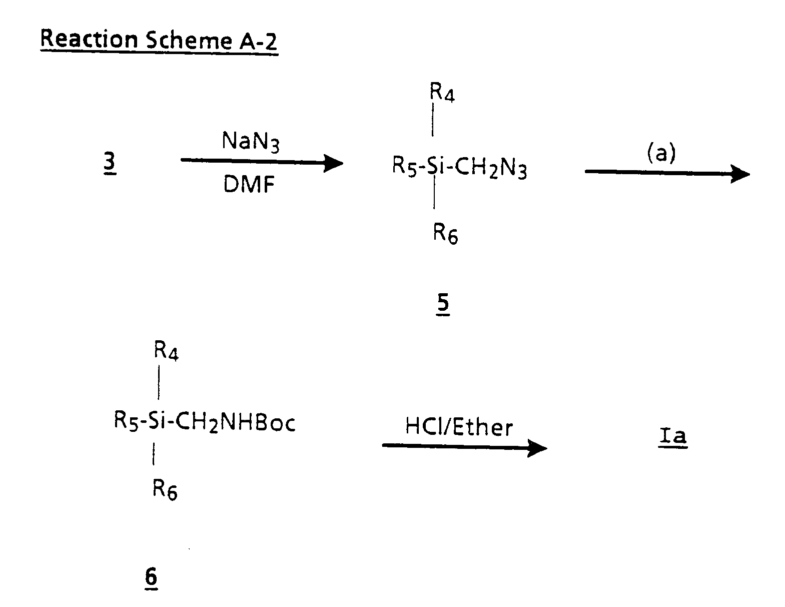
wherein (a) represents the sequential steps using (1) PØ₃, THF, Room Temp., (2) H₂O and (3) (BOC)₂O, THF; and R₄, R₅, and R₆ are as defined in formula I, and PØ₃ is triphenylphosphine.

In effecting the foregoing reaction (A-2) the formation of the azide is readily accomplished by standard reaction conditions for the displacement reaction, preferably by heating the reactants in an inert solvent (e.g., dry dimethylformamide) at 40°C. The conversion of the azide (5) to the corresponding amine (Ia) is effected through its N-Boc derivative (6) by the sequential treatment with (1) triphenylphosphine (PØ₃) at about room temperature in tetrahydrofuran (THF) (2) treatment with water followed by (3) purification of the desired product by the formation of its N-t-butoxycarbonyl derivative by reaction with (BOC)₂O in THF at room temperature. The N-Boc derivative is converted to its amine HCl salt (Ia) by reaction with gaseous HCl in diethylether (i.e., 3N to saturated HCl in diethylether) at room temperature.

In those instances wherein it is desired to prepare compounds of formula I wherein x is one, n and y are zero and R₃ is H (i.e., R₄R₅R₆Si(CH₂)₂NH₂), esters (7) derived from the appropriate silylchloride (2) are reduced to their corresponding alcohols (8), preferably with lithium aluminum hydride and the alcohols are converted to their corresponding phthalimides (9) using Mitsunobu reaction conditions (i.e., treatment of the alcohol with diethylazodicarboxylate, triphenyl phosphine and phthalimide). The resulting phthalimides (9) are hydrolized to the corresponding amine hydrochloride by sequential reaction with hydrazine hydrate and aqueous HCl as described in Reaction Scheme A. The esters (7) are prepared by alkylation of (2) with a metallo derivative (preferably zinc or sodium) of ethyl acetate according to standard and well known conditions. This series of reactions is depicted in the following reaction scheme. 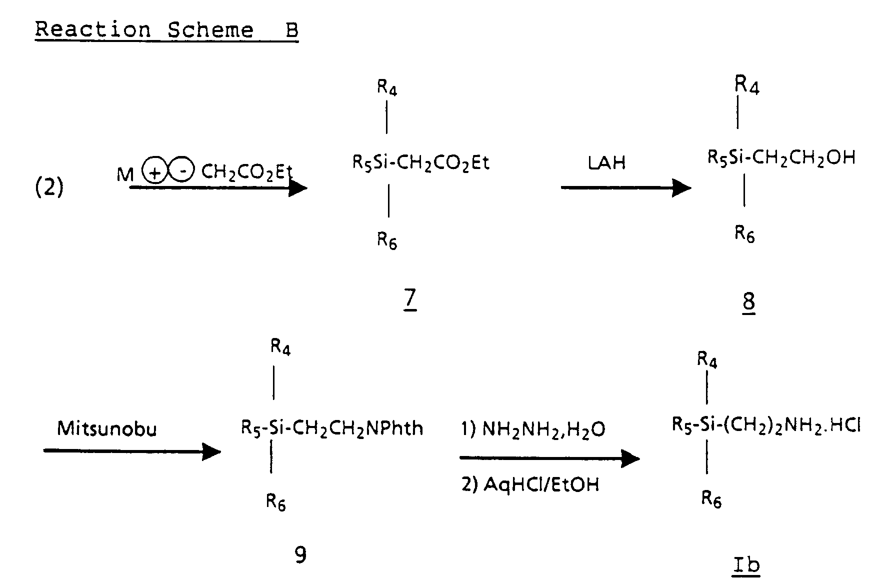
wherein M^{⊕} is a metallo cation of zinc or sodium, and R₄, R₅, R₆ and Nphth are as previously defined.

Alternatively, compounds (3) may be reacted with magnesium in diethylether to form the appropriate Grignard reagent which when treated with formaldehyde, (preferably using paraformaldehyde), will yield compounds of formula 8.

In those instances wherein it is desired to prepare compounds of formula I wherein x and y are zero and R₃ is other than H, it is convenient to subject tetra-chloro-silane (SiCl₄) to an alkylation with an organometallo Grignard-type reagent to obtain products (11) which are chlorinated to produce intermediate products (12). The chlorination is effected by standard techniques, preferably using sulfuryl chloride in the presence of a small amount of benzoyl peroxide as described by L.H. Sommer and F.C. Whitmore in JACS, 68, (1946) 485. The halogenated derivatives (12) are sequentially reacted with R₄MgX, R₅MgX and R₆MgX Grignard reagents to produce the appropriate R₄,R₅,R₆ substituted intermediates (13) by techniques known in the art such as, for example, by that taught by R.J. Fessenden and M.C. Coon in J. Med. Chem., 7, (1964) 561. Intermediates (13) are converted to the desired amines Id by procedures analogous to that described in Reaction Schemes A-1 and A-2. The foregoing reactions are well known in the art and are depicted by the following reaction scheme. 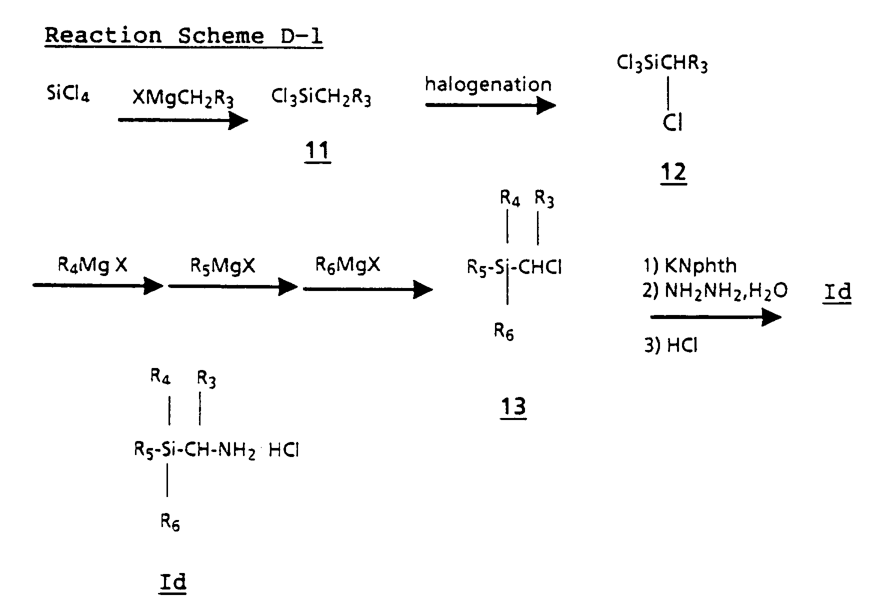
wherein X is halogen, R₄,R₅,R₆ and Nphth are as previously defined and R₃ is other than H.

Alternatively, as in Scheme A-2, compounds (13) may be converted to the corresponding azides by treatment with sodium azide and the azides converted to their amine HCl salts (Id) by sequential treatment with (1) triphenylphosphine (2) water and then converted to the corresponding N-Boc derivatives which, upon treatment with gaseous HCl in diethyl ether, are converted to the desired amines of formula (Id).

Another alternative process for preparing the compounds of formula (Id) is by the reaction of Scheme D-2. 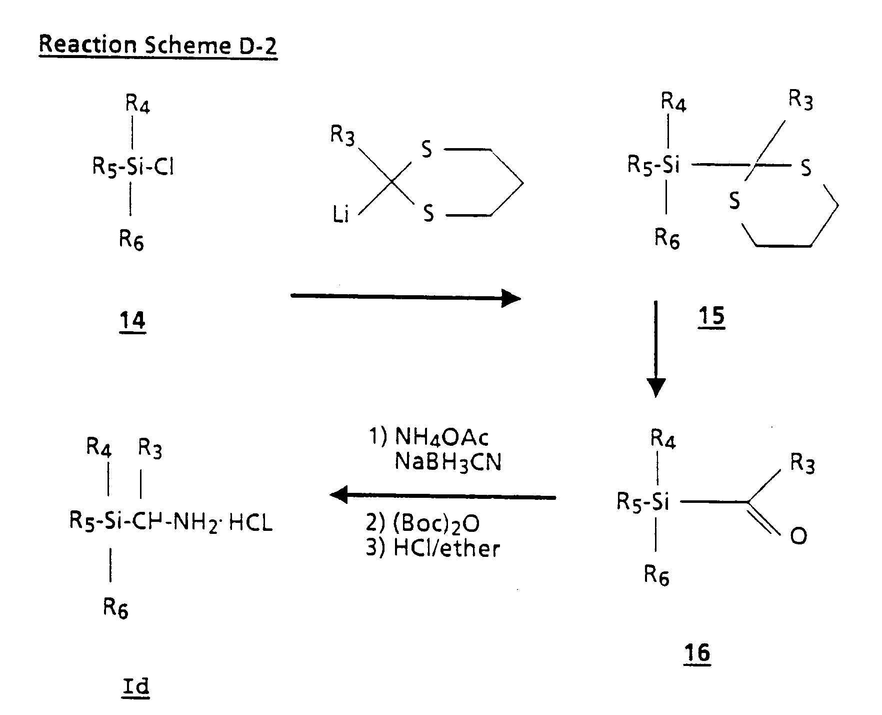
In effecting the foregoing reaction (Scheme D-2) the formation of the dithiane (15) is readily accomplished by standard reaction conditions for the condensation reaction, preferably by the procedure described in JACS 89, (1967), 434 by E.J. Corey, et al. Deprotection of the dithiane (15) to the corresponding ketone (16) is effected by reaction with mercuric chloride and mercuric oxide in a suitable solvent, preferably a mixture of methanol and water at reflux as described in JACS 89 (1967) 434 by E.J. Corey, et al. The ketone (16) is converted to the corresponding amine by reductive amination using sodium cyanoborohydride and ammonium acetate in a suitable solvent, preferably anhydrous methanol at room temperature. (JACS 93, (1971) 2897). The free amine is purified by conversion to its N-Boc derivative ((BOC)₂O, THF, RT). The N-Boc derivative is converted to the amine hydrochloride salt (Id) by reaction with HCl gas in diethyl ether (3N to saturated HCl/diethyl ether) at room temperature.

In those instances wherein it is desired to prepare compounds of formula I wherein x is zero, y is one and R₃ is other than hydrogen, it is convenient to react the chloromethylsilanes (13) with magnesium to produce the corresponding Grignard reagents (17) which are converted to their corresponding alcohols (18) by reaction with formaldehyde. These alcohols, (as analogously described for Reaction Scheme B) are subjected to the Mitsunobu conditions and hydrolysis reactions to produce intermediate phthalimides (19) and the desired amine hydrochlorides (Ie) respectively. The foregoing sequence of reactions is depicted by the following reaction scheme. 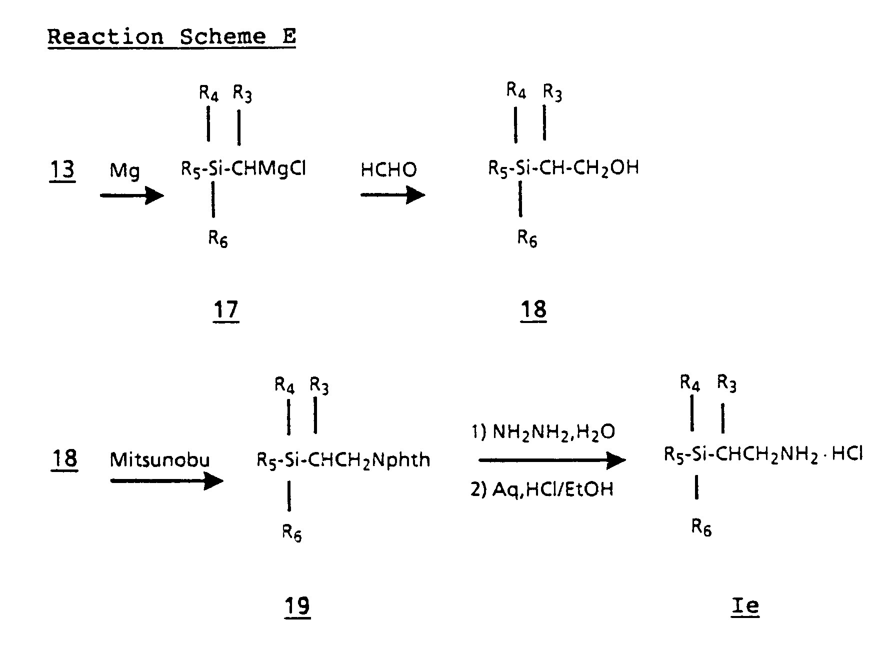
wherein R₄,R₅,R₆ and Nphth are as previously defined and R₃ is other than H.

In those instances wherein it is desired to prepare compounds of formula I wherein x is one, y is zero and R₃ is other than H, such compounds are conveniently prepared from silanes of formula 3 as the key starting materials. These silanes are alkylated with lithio derivatives of an appropriate R₃-substituted acetic acid t-butyl ester (20) and the resulting esters (21), after being treated with trifluoroacetic acid, are subjected to a Curtius Reaction (P.H.S. Smith, Organic Reactions III, 337 (1946)) which proceeds via the acyl azide (by reaction with NaN₃, acetone/water) to the isocyanate (by heating the azide at reflux in benzene) to its benzylcarbamate (by reacting the isocyanate with benzylalcohol) and to the desired amine (Ig) by hydrogenolysis (Pd/C, ethanol,H₂). This series of reactions is depicted by the following reaction scheme. 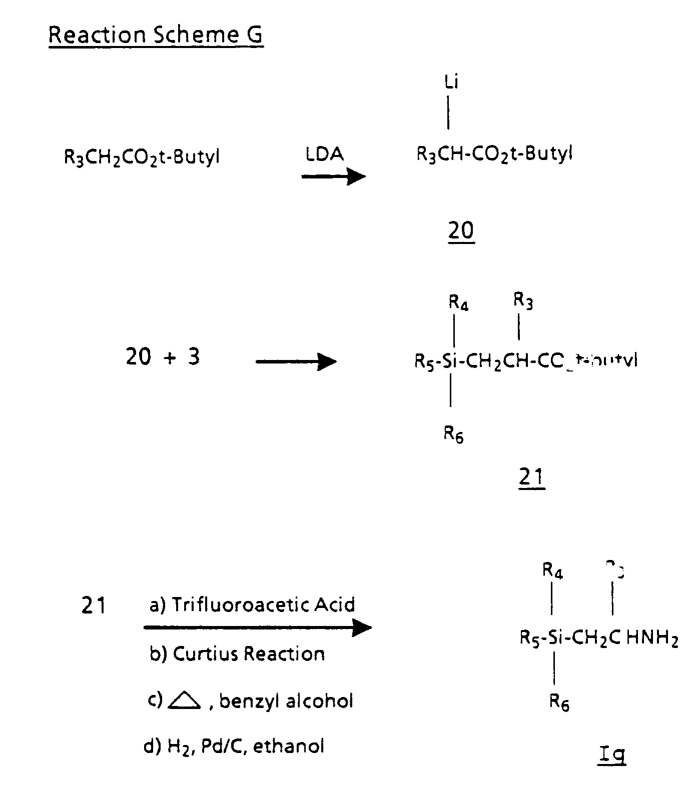
wherein R₄,R₅,R₆ are as previously defined and R₃ is other than H.

Alternatively, (using methodology analogous to Reaction Scheme E) compounds of formula 3 may be reacted with magnesium to produce their corresponding Grignards, which upon reaction with an R₃CHO reactant are transformed to their corresponding alcohol bearing an R₃ substituent (i.e., 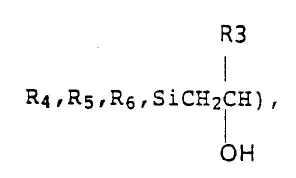
which are subjected to Mitsunobu conditions and hydrolysis to produce the appropriate phthalimides and amines (Ig) in the form of their hydrochloride salts.

The R₁ and/or R₂ substituted amines of the compounds of formula I may readily be prepared by reaction of the amine with any of the usual C₁₋₄ alkyl, benzyl, or phenethyl halides having a good S_{N}2 reactivity and the resulting hydrohalic salts neutralized according to procedures and techniques well known in the art. In certain cases, the amine may be reacted with an appropriate ketone (e.g., phenyl-2-propanone) in the presence of sodium cyano borohydride. Again these reactions are very well known and understood by those of ordinary skill in the art.

The following examples serve to illustrate the foregoing methods of synthesis and are not to be construed as limiting the generic sense of the invention herein described.

### EXAMPLE 1

### Benzyl-dimethylsilylmethanamine hydrochloride

**Step A.** N[(benzyl-dimethylsilyl)methyl]phthalimide
   Benzylchloromethyldimethylsilane⁽¹⁾ (0.420 g, 2.12 mmoles) and potassium phthalimide (0.430 g, 2.32 mmoles) were heated in dry dimethylformamide (DMF) (10 ml) at 70°C for 5 hours and then the mixture was poured into water and extracted with ether. The ether solution was washed with water, dried and evaporated to give a solid mass. Recrystallization from ether/hexane afforded N-[(benzyl-dimethylsilyl)methyl]phthalimide as white needles (0.620 g, 94% yield); mp 91-92°C; 1H NMR (CDCl₃, TMS) δ 0.07 (s,9H), 2.20 (s,2H), 3.17 (s,2H), 6.90-7.17 (m,5H), 7.50-7.87 (m,4H).
**Step B.** A mixture of N-[(benzyl-dimethylsilyl)methyl]phthalimide (0.620 g, 2 mmoles) and hydrazine hydrate (0.120 g, 2.4 mmoles) in ethanol (10 ml) was refluxed for 3 hours. 6N HCl (3 ml) was added and refluxing was continued for 30 min. Then the mixture was cooled, filtered and evaporated. Purification of the hydrochloride salt was achieved via its N-Boc derivative. Benzyl-dimethylsilylmethanamine hydrochloride was obtained as a white powder by cleavage of the N-protecting group using the method described in Step D of Example 5.(0.276 g, 64% yield); mp 138-139°C; TLC (n-BuOH-AcOH-H₂O, 6-2-2) Rf 0.55; 1H NMR (CDCl₃, TMS) δ 0.23 (s,6H), 2.31 (s,2H), 2.36 (s,2H), 7.23-7.76 (m,5H), 8.12 (broad s, 3H).

⁽¹⁾Fessenden, R.J., Coon, M.D., J. Med. Chem. Vol. 9, pp. 262-263 (1966).

| | | | |
|---|---|---|---|
| Anal.(C₁₀H₁₇NSi·HCl) C,H,N; Calc. | 55.67, | 8.41, | 6.49; |
| Found | 55.71, | 8.55, | 6.46. |

### EXAMPLE 2

### 2-(Trimethylsilyl)ethanamine·HCl

**Step A.** N-(2-Trimethylsilylethyl)phthalimide
A mixture of 2-trimethylsilylethanol (11.82 g, 0.1 mol) (commercially available), phthalimide (14.70 g, 0.1 mol), diethylazodicarboxylate (17.40 g, 0.1 mol) and triphenylphosphine (26.20 g, 0.1 mol) in anhydrous tetrahydrofuran (400 ml) was stirred, under nitrogen, at room temperature, for 16 hours. The solvent was removed in vacuo. The residue was taken off in benzene and the insoluble material was filtered off. The filtrate was evaporated to dryness. The residue was taken off in diethyl ether. The triphenylphosphine oxide was filtered off. The solvent was emoved in vacuo to yield 15.9 g of the expected pure N-(2-trimethylsilylethyl)phthalimide which was isolated by chromatography eluting from silica gel with ethyl acetate/cyclohexane 5/95. Rf is 0.63 using ethyl acetate/cyclohexane 1:1.
**Step B.** A mixture of N-(2-trimethylsilylethyl)-phthalimide (13.84 g, 55.9 mmol) and hydrazine hydrate (2.80 g, 55.9 mmol) in absolute ethanol (50 ml) was heated at reflux for 15 hours. The solvent was removed in vacuo. The residue was taken off in ethanol (50 ml) and 1N hydrochloric acid (56 ml) and heated at reflux for 5 hours. The solvent was removed in vacuo. The residue was taken off in cold water and the insoluble material was filtered off. The filtrate was washed with ethyl acetate (3x). The aqueous phase was evaporated to dryness yielding a yellow solid. Recrystallization from ethanol/diethyl ether yielded 6.41 g of pure 2-trimethylsilylethanamine hydrochloride.

| | | | | | | |
|---|---|---|---|---|---|---|
| Anal. calc. for C₅H₁₆NSiCl: | C%: | 39.07; | H%: | 10.49; | N%: | 9.11 |
| Found: | C%: | 39.05; | H%: | 9.95; | N%: | 9.11. |

### EXAMPLE 3

### β-(Benzyl-dimethylsilyl)ethanamine hydrochloride

**Step A.** β-(Benzyldimethylsilyl)ethanol
   To a solution of benzyldimethylsilylmethyl magnesium chloride, prepared from benzylchloromethyldimethyl silane (Fessenden, R.J., Coon, M.D., J. Med. Chem. Vol. 9 pp. 262-263 (1966)) (1.5 g, 7.55 mmol) and magnesium (0.19 g, 7.9 mmol) in diethyl ether (15 ml), is added paraformaldehyde (0.228 g, 7.6 mmol). Then the mixture is refluxed for 18 hours, cooled to 0°C and hydrolyzed with 1N HCl (25 ml). The organic layer is washed with water and brine and dried over MgSO₄. The solution is filtered and solvent removed *in vacuo*. Chromatography (silica gel, hexane/diethyl ether 70/30) afforded β-(benzyldimethylsilyl)ethanol as an oil (0.84 g, 57% yield).
   m.p. 132-133°C.
**Step B.** N-[β-(Benzyldimethylsilyl)ethyl]phthalimide
   A mixture of β-(benzyldimethylsilyl)ethanol (0.84 g, 4.33 mmol), phthalimide (0.66 g, 4.5 mmol), diethylazodicarboxylate (0.783 g, 4.5 mmol) and triphenylphosphine (1.18 g, 4.5 mmol) in anhydrous tetrahydrofuran (80 ml) is stirred, under nitrogen, at room temperature for 18 hours. The solvent is removed in vacuo. The residue is taken off in toluene and the insoluble material is filtered off. The filtrate is evaporated to dryness. The residue is taken off in diethyl ether and the insoluble material is filtered off. The solvent is removed *in vacuo*. Chromatography (silica gel, hexane/diethyl ether - 80/20) affords the title compound (0.94 g, 67% yield).
**Step C.** β-(Benzyldimethylsilyl)ethanamine hydrochloride
   The title compound is prepared in 71% yield by a procedure similar to the one described in Example 8, Step C.
   m.p. 132-133°C.

### EXAMPLE 4

### β-(Dimethyl-2-phenylethylsilyl)ethanamine hydrochloride

**Step A.** Chloromethyldimethyl-2-phenylethylsilane
   To a solution of 2-phenylethylmagnesium bromide, prepared from 2-phenylethyl bromide (9.25 g, 50 mmol) and magnesium (1.34 g, 55 mmol) in tetrahydrofuran (50 ml), is added chlorochloromethyldimethylsilane (6.43 g, 45 mmol) in tetrahydrofuran (25 ml). The mixture is refluxed for 18 hours, cooled to 0°C and hydrolyzed with 3N HCl (50 ml). The mixture is then poured in water (50 ml) and diethyl ether (50 ml), the organic layer is washed with water, brine, dried over MgSO₄ and the solvents removed *in vacuo*. Fractional distillation affords the title compound (5.15 g, 48.5% yield).
   b.p. 113-115°C (5 mmHg).
**Step B.** β-(Dimethyl-2-phenylethylsilyl)ethanol
   The compound of this step is prepared in 58% yield by a procedure similar to the one described in Example 3, Step A.
**Step C.** β-N-[(Dimethyl-2-phenylethylsilyl)ethyl]-phthalimide
   The compound of this step is prepared in 76% yield by a procedure similar to the one described in Example 3, Step B.
**Step D.** β-(Dimethyl-2-phenylethylsilyl)ethanamine hydrochloride
   The title compound (m.p. 137-138°C) is prepared in 60% yield from the product of Step C by a procedure similar to the one described in Example 8, Step C.

### EXAMPLE 5

### α-(Trimethylsilyl)benzeneethanamine·HCl

**Step A.** 2-Benzyl-2-trimethylsilyl-1,3-dithiane
The title compound of this step was prepared in 75% yield, by the procedure described in JACS 89, (1967), 434 by E.J. Corey, et al.,using 2-benzyl-1,3-dithiane and trimethylsilyl chloride. The product was recrystallized from diethyl ether/pentane.
**Step B.** Benzyl-trimethylsilylketone
The title compound of this step was prepared in 73% yield by hydrolysis of 2-benzyl-2-trimethylsilyl-1,3-dithiane using mercuric chloride and mercuric oxide, following the procedure described in JACS 89, (1967), 434, by E.J. Corey, et al. The product was purified by Kugelrohr distillation, Bp (Kugelrohr) 170°C/16 mmHg.
**Step C.** N-t-butoxycarbonyl-α-(trimethylsilyl)-benzeneethanamine
A mixture of benzyl-trimethylsilylketone (1.40 g, 7.3 mmol), sodium cyanoborohydride (0.321 g, 5.1 mmol) and ammonium acetate (5.93 g, 73 mmol) in anhydrous methanol (25 ml) was stirred at room temperature for 48 hours. The mixture was poured into 2N sodium hydroxide (20 ml) and extracted with diethyl ether (3 x 100 ml). The organic phase was dried over anhydrous magnesium sulphate. Trituration, removal of the solvent, *in vacuo*, and chromatography (from silica gel, ethyl acetate/cyclohexane 1:1) yielded the crude α-(trimethylsilyl)benzeneethanamine which was directly converted to the product of this step using 1 equivalent of (BOC)₂O in THF. Rf: 0.74(ethyl acetate/cyclohexane 1:1) mp: 67-68°C.
**Step D.** A 3N solution of HCl in diethyl ether (3 ml) was added at room temperature to a mixture of N-t-butoxycarbonyl-α-(trimethylsilyl)benzeneethanamine(0.150g, 0.51 mmol) in diethyl ether (3 ml). The mixture was stirred at room temperature for 48 hours. The resulting solid was filtered off and dried, *in vacuo*, to yield 0.106 g of the expected α-(trimethylsilyl)-benzeneethanamine hydrochloride, mp 193°C.

| | | | | | | |
|---|---|---|---|---|---|---|
| Anal. calc. for C₁₁H₂₀NSiCl | C%: | 57.49; | H%: | 8.77; | N%: | 6.09. |
| Found | C%: | 57.40; | H%: | 8.56; | N%: | 6.01. |

Rf: 0.61(AcOH/BuOH/H₂O,2-6-2).

### Example 6

### α-(Trimethylsilylmethyl)benzenemethanamine hydrochloride

**Step A.** N-Benzyloxycarbonyl-α-(trimethylsilylmethyl)benzenemethanamine
A mixture of α-(trimethylsilylmethyl)benzeneethanoic acid (4.80 g, 21.6 mmol) and thionyl chloride (40 ml) was heated at reflux for 2 hours. The solvent was removed in vacuo. The crude acyl chloride was dissolved in acetone (35 ml) to which was added a solution of sodium azide (1.55 g, 23.8 mmol) in water (3 ml) at 0°C. The mixture was stirred for 1 hour at that temperature. Water was added and the mixture was extracted with diethyl ether. The organic phase then was dried over anhydrous magnesium sulphate. Filtration and removal of the solvent *in vacuo* yielded the expected acyl azide. The crude acyl azide was taken off in benzene and the solution was heated at reflux for 12 hours. The solvent was then evaporated *in vacuo* to yield the expected isocyanate. The isocyanate was taken off in dichloromethane and reacted with benzyl-alcohol (1 equiv.). The mixture was heated at reflux for 15 hours. The solvent was removed *in vacuo*. Chromatography (silica gel, ethyl acetate/cyclohexane 5:95) yielded the pure N-benzyloxycarbonyl-α-(trimethylsilylmethyl)benzene-methanamine in 50% overall yield, which is recrystallized from diethyl ether/pentane.

| | | | | | | |
|---|---|---|---|---|---|---|
| Anal. Calc. for C₁₉H₂₅NO₂Si: | C%: | 69.68; | H%: | 7.69; | N%: | 4.28. |
| Found: | C%: | 69.81; | H%: | 7.63; | N%: | 4.26. |

**Step B.** α-(Trimethylsilylmethyl)benzenemethanamine·HCl
A mixture of N-benzyloxycarbonyl-α-(trimethylsilylmethyl)benzenemethanamine (2.44 g, 7.5mmol) and 10% palladium on charcoal (0.25 g) in ethanol (90 ml) was stirred at room temperature under a hydrogen atmosphere for 15 hours. Filtration of the catalyst and removal of the solvent *in vacuo* yielded α-(trimethyl-silylmethyl)-benzenemethanamine in 70% yield as a white solid. A 3N HCl/diethyl ether solution (3.7 ml) was added at 0°C, to a solution of α-(trimethylsilylmethyl)benzenemethanamine (0.71 g, 3.7 mmol) in absolute ethanol (5 ml). The temperature was allowed to rise to room temperature overnight. Diethyl ether was added to precipitate the expected α-(trimethylsilylmethyl)benzenemethanamine hydrochloride which was isolated by filtration in 65% yield.

| | | | | | | |
|---|---|---|---|---|---|---|
| Anal. Calc. for: C₁₁H₂₀NSiCl: | C%: | 57.49; | H%: | 8.77; | N%: | 6.09. |
| Found: | C%: | 57.08; | H%: | 9.09; | N%: | 6.07. |

mp: 228°C.

### EXAMPLE 7

### Ethyl-4-fluorobenzylmethylsilylmethanamine hydrochloride

**Step A.** Chlorochloromethylethylmethylsilane
   A solution of ethylmagnesium bromide, prepared from ethylbromide (2.18 g, 20 mmol) and magnesium (0.49 g, 20 mmol) in diethyl ether (20 ml), is added dropwise at 0°C to a solution of chloromethyldichloromethyl silane (3.27 g, 20 mmol) in diethyl ether (5 ml). The reaction is then refluxed for 18 hours, cooled and filtered under argon. The filtrate is concentrated by distillation at atmospheric pressure. Fractional distillation affords the title compound (2.04 g, 65.4% yield).
   b.p. 125°C (760 mmHg).
**Step B.** Chloromethylethyl-4-fluorobenzylmethylsilane
   The compound of this step is prepared in 89% yield from the product of Step A by a procedure similar to the one described in Example 8, Step A.
**Step C.** N-[(Ethyl-4-fluorobenzylmethylsilyl)methyl]-phthalimide
   The compound of this step is prepared in 36% yield from the product of Step B by a procedure similar to the one described in Example 8, Step B.
**Step D.** Ethyl-4-fluorobenzylmethylsilylmethanamine hydrochloride
   The title compound is prepared in 38% yield from the product of Step C by a procedure similar to the one described in Example 8, Step C. m.p. 138°C.

### EXAMPLE 8

### Dimethyl-4-fluorobenzylsilylmethanamine·hydrochloride

**Step A.** Chloromethyl-dimethyl-4-fluorobenzylsilane
   To the 4-fluorobenzylmagnesiumchloride, prepared from 4-fluorobenzylchloride (1.59 g, 11 mmoles) and magnesium (0.25 g, 11 mmoles) in ether (20 ml), was added dropwise, at 0°C, chloro-chloromethyl-dimethylsilane (1.43 g, 10 mmoles) in ether (5 ml). Then the mixture was refluxed 6 hours, cooled to 0°C, hydrolyzed with 3N HCl (20 ml) and extracted with ether. The ether solution was washed with water, brine, dried and evaporated. Chromatography (silica gel, hexane) afforded chloromethyl-dimethyl-4-fluorobenzyl silane as an oil (2.01 g, 93% yield) 1H NMR (CDCl₃, TMS) δ:0.20 (s,6H), 2.27 (s,2H), 2.63 (s,2H), 6.90-7.13 (m,4H).
**Step B.** N-[(dimethyl-4-fluorobenzylsilyl)methyl]-phthalimide
   Chloromethyl-dimethyl-4-fluorobenzylsilane (2.01 g, 9.33 mmoles) and potassium phthalimide (1.90 g), 10.27 mmoles) were heated in dry dimethylformamide (30 ml) at 70°C for 5 hours and then the mixture was poured into water and extracted with ether. The ether solution was washed with water, brine, dried and evaporated. Chromatography (silica gel, ether/hexane-20/80) afforded N-[(dimethyl-4-fluorobenzylsilyl)methyl]phthalimide as a white powder (2.90 g, 95% yield) 1H NMR (CDCl₃, TMS) δ:0.20 (s,6H), 2.25 (s,2H), 3.27 (s,2H), 6.83-7.13 (m,4H), 7.67-7.97 (m,4H).
**Step C.** Dimethyl-4-fluorobenzylmethanamine hydrochloride
   A mixture of N-[(dimethyl-4-fluorobenzylsilyl)methyl]-phthalimide (2.90 g, 8.88 mmoles) and hydrazine hydrate (0.53 g, 10.66 mmoles) in ethanol (30 ml) was refluxed for 3 hours. 6N HCl (7 ml) was added and refluxing was continued for 30 minutes. Then the mixture was cooled, filtered and evaporated. Purification of the hydrochloride salt was achieved via its N-Boc derivative. Dimethyl-4-fluorobenzylmethanamine hydrochloride was obtained as a white powder by cleavage of the N protecting group using the method described in Step D of Example 5. (1.25 g, 60% yield) mp 134-135°C 1H NMR (CDCl₃, TMS) δ : 0.20 (s,6H), 2.27 (s,2H), 2.30-2.57 (m,2H), 6.77-7.00 (m,4H), 8.07 (broad s, 3H).

### EXAMPLE 9

### 2-Trimethylsilyl propanamine·hydrochloride

**Step A.** 2-Trimethylsilyl propanol
   To a solution of α-trimethylsilylethylmagnesium chloride [prepared from (α-chloroethyl)trimethylsilane (L.H. Sommer, F.C. Whitmore, JACS 68, (1946) 485)(1.370 g, 10 mmol) and magnesium (0.27 g, 11 mmoles) in diethyl ether (20 ml)] was added paraformaldehyde (0.30 g). The mixture was refluxed for 18 hours, cooled to 0°C and hydrolysed with 1N HCl (30 ml). The organic layer was washed with water, brine and dried over MgSO₄. Filtration and evaporation left an oil. Chromatography (silica gel, hexane/diethyl ether 70/30) afforded 2-trimethylsilyl-propanol.
**Step B.** N-[(2-Trimethylsilyl)propyl]phthalimide
   The title compound was prepared from 2-trimethylsilylpropanol by a procedure similar to the one described in Example 2, Step A.
**Step C.** 2-Trimethylsilylpropanamine · hydrochloride
   The title compound was prepared from N-[(2-trimethylsilyl)propyl]phthalimide by a procedure similar to the one described in Example 2, Step B.

### EXAMPLE 10

### Dimethyl-3-fluorobenzylsilylmethanamine hydrochloride

**Step A.** Chloromethyldimethyl-3-fluorobenzylsilane
   The compound of this step is prepared in 93% yield by a procedure similar to the one described in Example 8, Step A.
**Step B.** Acetoxymethyldimethyl-3-fluorobenzylsilane
   Chloromethyldimethyl-3-fluorobenzylsilane (0.85 g, 3.93 mmol) and potassium acetate (0.83 g, 8.45 mmol) are refluxed in acetic acid (10 ml) for 48 hours. The mixture is then cooled, poured into water (50 ml) and extracted with diethyl ether (50 ml). The organic layer is washed with water, brine, dried over MgSO₄ and the solvent is removed *in vacuo*. Chromatography (silica gel, hexane/ether - 80/20) affords the title compound as an oil (0.38 g, 40% yield).
**Step C.** Dimethyl-3-fluorobenzylhydroxymethylsilane
   To a mixture of lithium aluminum hydride (0.06 g, 1.58 mmol) in diethyl ether (5 ml) is added dropwise acetoxymethyldimethyl-3-fluorobenzylsilane (0.38 g, 1.58 mmol) in diethyl ether (2 ml). The mixture is then allowed to react for 0.5 hours at room temperature, treated with ethylacetate (2 ml), hydrolyzed with 1N HCl (6.5 ml), and poured into water (20 ml) and diethyl ether (10 ml). The organic layer is washed with water, brine, dried over MgSO₄ and the solvent removed *in vacuo*. Chromatography (silica gel, hexane/ether - 60/40) affords the title compound (0.23 g, 73% yield).
**Step D.** N-[(Dimethyl-3-fluorobenzylsilyl)methyl]phthalimide
   The compound of this step is prepared in 69% yield from the product of step C by a procedure similar to the one described in Example 8, Step B.
**Step E.** Dimethyl-3-fluorobenzylsilylmethanamine hydrochloride
   The title compound is prepared in 35% yield from the product of Step D by a procedure similar to the one described in Example 8, Step C., m.p. 107°C.

### EXAMPLE 11

### α-(Benzylethylmethylsilyl)ethanamine · hydrochloride

**Step A.** (α-Chloroethyl)trichlorosilane
   The title compound was prepared from ethyltrichlorosilane (Kipping, J. Chem. Soc. 91 (1907) 214) by the method described by L.H. Sommer, F.C. Whitmore, JACS 68 (1946), 485.
**Step B.** (α-Chloroethyl)dichloromethylsilane
   To a solution of (α-chloroethyl)trichlorosilane (1.980 g, 10 mmol) in diethyl ether (100 ml) was added a 3M solution of methylmagnesium bromide (3.3 ml). The mixture was refluxed for 12 hours, then filtered. The product was purified by distillation.
**Step C.** (α-Chloroethyl)chloroethylmethylsilane
   To a solution of (α-chloroethyl)dichloromethylsilane (0.355 g, 2 mmol) in diethyl ether (10 ml) was added a solution of ethylmagnesium bromide (2 mmol, 1 eq). The mixture was refluxed for 12 hours, then filtered. The product was purified by distillation.
**Step D.** (α-Chloroethyl)benzylethylmethylsilane
   To a solution of (α-chloroethyl)chloroethylmethylsilane (0.171 g, 1 mmol) in diethyl ether (5 ml) was added a solution of benzylmagnesium bromide (1 mmol, 1 eq). The mixture was refluxed for 12 hours, then filtered. The product was purified by distillation.
**Step E.** N-[1-(Benzylethylmethylsilyl)ethyl]-phthalimide
   The title compound was prepared from (α-chloroethyl)benzylethylmethylsilane and potassium phthalimide by a procedure similar to the one described in Example 1, Step A.
**Step F.** α-(Benzylethylmethylsilyl)ethanamine · hydrochloride
   The title compound was prepared from N-[1-(benzylethylmethylsilyl)ethyl]phthalimide by a procedure similar to the one described in Example 1, Step B.

### EXAMPLE 12

### 3,4-Difluorobenzyldimethylsilylmethanamine·hydrochloride

**Step A.** Chloromethyl-3,4-difluorobenzyldimethylsilane
   The compound of this step is prepared in 97% yield by a procedure similar to the one described in Example 8, Step A.
**Step B.** N-[(3,4-difluorobenzyldimethylsilyl)methyl]phthalimide
   The compound of this step is prepared in 56% yield from the product of Step A by a procedure similar to the one described in Example 8, Step B.
**Step C.** 3,4-Difluorobenzyldimethylsilylmethanamine hydrochloride
   The title compound is prepared from the product of Step B by a procedure similar to the one described in Example 8, Step C. m.p. 132°C.

### EXAMPLE 13

### 2,6-Difluorobenzyldimethylsilylmethanamine hydrochloride

**Step A.** Chloromethyl-2,6-difluorobenzyldimethylsilane
   The title compound is prepared in 74% yield by a procedure similar to the one described in Example 8, Step A.
**Step B.** N-[(2,6-Difluorobenzyldimethylsilyl)methyl]phthalimide
   The compound of this step is prepared in 52% yield from the product of Step A by a procedure similar to the one described in Example 8, Step B.
**Step C.** 2,6-Difluorobenzyldimethylsilylmethanamine hydrochloride
   The title compound is prepared in 44% yield from the product of Step B by a procedure similar to the one described in Example 8, Step C. m.p. 151-152°C.

### EXAMPLE 14

### 2,4-Difluorobenzyldimethylsilylmethanamine·hydrochloride

**Step A.** Chloromethyl-(2,4-difluorobenzyl)dimethylsilane
   The title compound is prepared in 95% yield from 2,4-difluorobenzylmagnesium chloride and chlorochloromethyldimethylsilane by the method described in Example 8, Step A.
**Step B.** (2,4-Difluorobenzyl)dimethylsilylmethanazide
   A mixture of chloromethyl-(2,4-difluorobenzyl)dimethylsilane (11.40 g, 48.6 mmol) and sodium azide (12.64 g, 194.5 mmol) in anhydrous dimethylformamide (240 ml) is heated at 40°C for 18 hours. Water is added to the mixture. Extraction with diethyl ether and the standard workup afforded 9.40 g of the desired compound (80% yield). TLC: Rf 0.59 (silica gel, ethyl acetate/cyclohexane 2:8).
**Step C.** N-tert-butoxycarbonyl-(2,4-difluorobenzyl)dimethylsilylmethanamine
   A mixture of (2,4-difluorobenzyl)dimethylsilylmethanazide (4.65 g, 19.3 mmol) and triphenylphosphine (5.06 g, 19.3 mmol) in anhydrous tetrahydrofuran (80 ml) is stirred at room temperature, under nitrogen, for 4 hours. Water (0.52 ml) and di-t-butyldicarbonate (4.21 g, 19.3 mmol) are added and the mixture stirred at room temperature, under nitrogen for 18 hours. Water (40 ml) is added and the mixture is extracted with diethyl ether (3 x 80 ml). The organic layer is dried over anhydrous magnesium sulfate. Filtration and removal of the solvent in vacuo leaves an oil which is purified by chromatography (silica gel, cyclohexane). 1.89 g of the desired compound is thus obtained (30% yield). TLC: Rf 0.43 (silica gel, ethyl acetate/cyclohexane 2:8).
**Step D.** (2,4-difluorobenzyl)dimethylsilylmethanamine·hydrochloride
   The title compound is obtained in 90% yield from N-tert-butoxycarbonyl(2,4-difluorobenzyl)dimethylsilylmethanamine by the procedure described in Example 5, Step D. m.p.: 144°C.

### EXAMPLE 15

### Dimethyl-2-fluorobenzylsilylmethanamine·hydrochloride

**Step A.** Chloromethyldimethyl-2-fluorobenzylsilane
   The compound of this step is prepared in 67% yield by a procedure similar to the one described in Example 8, Step A.
**Step B.** N-[Dimethyl-2-fluorobenzylsilylmethyl]phthalimide
   The compound of this step is prepared in 29% yield from the product of Step A by a procedure similar to the one described in Example 8, Step B
**Step C.** Dimethyl-2-fluorobenzylsilylmethanamine.-hydrochloride
   The title compound is prepared in 22% yield from the product of Step B by a procedure similar to the one described in Example 8, Step C, m.p. 148°C.

Having generically described the methods of synthesis for the preparation of the compounds of Formula I and also having illustrated the generic teachings with the foregoing specific examples, it is obvious to one of ordinary skill in the art that by the appropriate modifications in the reactants, the following compounds readily will be produced:
benzyldiethylsilylmethanamine,
benzylethylmethylsilylmethanamine,
4-chlorobenzyldimethylsilylmethanamine,
4-fluorobenzyldimethylsilylmethanamine,
dimethyl-4-methoxybenzylsilylmethanamine,
dimethyl-4-trifluoromethylbenzylsilylmethanamine,
dimethyl-4-hydroxybenzylsilylmethanamine,
dimethyl-4-cyanobenzylsilylmethanamine,
dimethyl-4-nitrobenzylsilylmethanamine,
3,4-difluorobenzyldimethylsilylmethanamine,
ethylmethyl-3-methyl-4-fluorobenzylsilylmethanamine,
ethylmethyl-4-fluorophenethylsilylmethanamine,
dimethyl-(3-pyridylmethyl)silylmethanamine,
dimethyl-(3-indolylmethyl)silylmethanamine,
dimethyl-(β-(5-hydroxy-3-indolyl)ethyl)silylmethanamine,
(2,4-difluorobenzyl)methylethylsilylmethanamine,
(2,4-difluorobenzyl)dimethylethylsilylmethanamine,
(2,4-difluorophenethyl)-dimethylsilylmethanamine,
(3,4-dichlorobenzyl)-dimethylsilylmethanamine,
(3,4-difluorobenzyl)-methylethylsilylmethanamine,
dimethyl-(2-furylmethyl)silylmethanamine,
dimethyl-(2-thienylmethyl)silylmethanamine,
dimethyl-(3-indazolylmethyl)silylmethanamine.
as well as the corresponding silylethanamine analogs and
2-triethylsilyl-2-benzylethanamine,
1-trimethylsilyl-benzylmethanamine,
1-trimethylsilyl-1-(4-chlorobenzyl)methanamine,
2-trimethylsilyl-2-(4-chlorobenzyl)ethanamine,
2-trimethylsilyl-2-(4-methoxybenzyl)ethanamine,
2-trimethylsilyl-2-(4-trifluoromethylbenzyl)ethanamine,
2-triethylsilyl-2-(4-hydroxybenzyl)ethanamine,
2-trimethylsilyl-2-(4-cyanobenzyl)ethanamine,
2-trimethylsilyl-2-(4-nitrobenzyl)ethanamine,
2-triethylsilyl-2-(3,4-difluorobenzyl)ethanamine,
2-trimethylsilyl-2-(3-pyridylmethyl)ethanamine,
2-triethylsilyl-2-(3,4-difluorophenyl)ethanamine,
2-triethylsilyl-2-(3-indolylmethyl)ethanamine.

In its method-of-use aspect this invention relates to the use of compounds of the formula 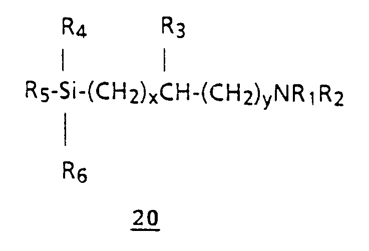
and the pharmaceutically acceptable salts thereof, wherein each of x and y is zero or one, with the proviso that the sum of x and y is less than two,
- R₁: is H or C₁₋₁₀ alkyl,
- R₂: is H, C₁₋₁₀ alkyl, (CH₂)ₘ phenyl with m being 1 to 4,
- R₃: is H or (CH₂)ₙR₃' with n being zero or 1 to 4,
- R₃': is C₁₋₁₀ alkyl, or aryl;
- R₄: is C₁₋₁₀ alkyl,
- R₅: is (CH₂)ₘR₅' with m being 1 to 4,
- R₆: is (CH₂)ₚR₆' with p being 1 to 4, with R₃', R₅' and R₆' being H, C₁₋₁₀ alkyl, aryl or x,y substituted aryl, each of x and y being C₁₋₁₀ alkyl, C₁₋₁₀ alkoxy, CF₃, halogeno, OH, CN or NO₂, with the proviso that R₃ is other than hydrogen when each of R₄, R₅ and R₆ is methyl,
for the treatment of Parkinson's Disease.

The class of compounds known as monoamine oxidase inhibitors (MAO inhibitors) has been employed in psychiatry for over 20 years for the treatment of depression, (See Goodman and Gilman, *The Pharmacological Basis of Therapeutics*, 6th Ed., McMillan Publishing Co., Inc., N.Y., 1980, pages 427-430). MAO inhibitors currently used in the USA for treating depression are tranylcypromine (PARNATE, SKF), phenelzine (NARDIL, Parke-Davis), and isocarboxazid (MARPLAN, Roche). In addition, another MAO inhibitor, pargyline (EUTRON, Abbott) is available for the treatment of hypertension [See *Physicians' Desk Reference*, 34th Ed., Medical Economics Co., Oradell, N.J., 1980, pages 1327-1328 (phenelzine), pages 1466-1468 (isocarboxazid), pages 1628-1630 (tranylcypromine) and pages 521-522 (pargyline)]. In addition to being used in treating depression, MAO inhibitors can be employed to treat other psychiatric disorders, such as phobic anxiety states.

It is believed that the MAO inhibitors act to alleviate psychiatric disorders, such as depression, by increasing the concentration of one or more biogenic monoamines in the brain or sympathetic nervous system. The monoamine oxidase enzyme (MAO) plays an important role in the metabolic regulation of the monoamines since it catalyzes the biodegradation of the monoamines through oxidative deamination. By inhibiting MAO, the degradation of the monoamines is blocked, and the result is an increase in the availability of the monoamines for their physiological functions. Among the physiologically active monoamines which are known substrates for MAO are: (a) the so-called "neurotransmitter" monoamines, such as the catecholamines (e.g., dopamine, epinephrine, and norepinephrine) and the indoleamines (e.g., tryptamine and 5-hydroxytryptamine), (b) the so-called "trace" amines (e.g., o-tyramine, phenethylamine, tele-N-methylhistamine), and (c) tyramine.

The usefulness of the MAO inhibitors in treating depression is limited because the administration of such agents can potentiate the pharmacological actions of certain food substances or drugs leading to dangerous and sometimes lethal effects. For example, persons receiving a MAO inhibitor must avoid the ingestion of foods which have a high tyramine content (such as cheese) because the MAO inhibitor will block the metabolic degradation of tyramine in the gut to produce high circulating levels of tyramine, consequent release of catecholamines in the periphery, and finally serious hypertension. The potentiation by a MAO inhibitor of the pressor effect of tyramine arising from the ingestion of cheese, and the hypertensive episode produced thereby, are commonly known as the "cheese reaction" or "cheese effect". Moreover, persons on conventional MAO therapy can not be given directly-acting sympathomimetic drugs (or precursors thereof) which are themselves substrates for MAO (e.g., dopamine, epinephrine, norepinephrine, or L-DOPA) and indirectly-acting sympathomimetic drugs (e.g., amphetamines or cold, hay-fever, or weight control preparations that contain a vasoconstrictor). The potentiation of the pressor effects of indirectly-acting sympathomimetic drugs is especially profound. This is because such drugs act peripherally primarily by releasing catecholamines in nerve endings, and the concentration of the liberated catecholamines will be dangerously elevated if the metabolic degradation of the catecholamines via MAO is blocked.

Biochemical and pharmacological studies indicate that the MAO enzyme exists in two forms known as "MAO Type A" (MAO-A) and "MAO Type B" (MAO-B). The two forms differ in their distribution in body organs, in their substrate specificity, and in their sensitivity to inhibitors. In general, MAO-A selectively oxidizes the so-called "neuro-transmitter" monoamines (epinephrine, norepinephrine, and 5-hydroxytryptamine) while MAO-B selectively oxidizes the "trace" monoamines (o-tyramine, phenethylamine, and tele-N-methylhistamine). Both MAO-A and MAO-B oxidize tyramine, tryptamine, and dopamine. However, in man, dopamine has been shown to be a preferred substrate for MAO-B. The forms also differ in their sensitivity to inhibition, and thus they can be preferentially inhibited depending upon the chemical structure of the inhibitor and/or the relative concentrations of the inhibitor and the enzyme. The MAO inhibitors currently sold in the USA for the therapy of depression (tranylcypromine, phenelzine, and isocarboxazid) are not preferential in the action upon MAO. However, various chemical compounds are known in the art to be preferential inhibitors of MAO, the most important being clorgyline, pargyline, and L-deprenyl which are all reported to be clinically effective antidepressant agents. MAO-A is preferentially inhibited by clorgyline, while MAO-B is preferentially inhibited by pargyline and L-deprenyl. It should be observed that the "selectivity" of a MAO inhibitor arises because the inhibitor has a greater affinity for one form of the enzyme. Thus, the selectivity of an inhibitor for MAO-A or MAO-B in vivo will be dose-dependent, selectivity being lost as the dosage is increased. Clorgyline, pargyline, and L-deprenyl are selective inhibitors at lower dosages, but are not selective inhibitors at higher dosages. The literature concerning MAO-A and MAO-B, and the selective inhibition thereof, is extensive. [See, for example, Goodman and Gilman, ibid, pages 204-205; Neff et al, Life Sciences, 14, 2061 (1974); Murphy, *Biochemical Pharmacology*, 27, 1889 (1978); Knoll, Chapter 10, pages 151-171 and Sandler, Chapter 11, pages 173-181, in *Enzyme Inhibitors as Drugs*, M. Sandler, Ed., Macmillan Press Ltd., London, 1980; Lipper et al, *Psychopharmacology*, 62, 123 (1979); Mann et al, Life Sciences, 26, 877 (1980); and various articles in *Monoamines Oxidase: Structure, Function and Altered Functions*, T. Singer et al Ed., Academic Press, N.Y., 1979].

Of the selective inhibitors of MAO, L-deprenyl is of interest since the "cheese effect" is not observed at the low dosages where preferential inhibition of MAO-B occurs. [See Knoll, TINS, pages 111-113, May 1979]. This observation is not unexpected since the intestinal mucosa contains predominantly MAO-A which, because it is not inhibited, permits oxidation and removal of the ingested tyramine. The selectivity of L-deprenyl for MAO-B may account for its ability to potentiate L-DOPA for the treatment of Parkinson's disease without producing peripheral side effects, such as hypertension due to potentiation of pressor catecholamines [See Lees et al, Lancet, pages 791-795, Oct. 15, 1977 and Birkmeyer, Lancet, pages 439-443, Feb. 26, 1977].

The compounds of formula I are pharmacologically active, being capable of inhibiting MAO as by demonstration in standard biological test procedures performed *in vitro* or *in vivo* in laboratory animals. Indeed, based upon standard laboratory procedures, the compounds of formula I are potent and selective inhibitors of MAO-B; the inhibition being time-dependent, active site directed and irreversible. The compounds, in general, will exert their MAO inhibition within the dose range of 0.01 to 10 mg per kilogram of body weight. In particular the compounds of formula I are capable of preferentially inhibiting the B form of MAO *in vitro*, and *in vivo*, such compounds will inhibit MAO-B without substantially inhibiting MAO-A. Thus at such dosages wherein they exert a selective effect on MAO-B the compounds will not exert a marked "cheese effect". Therefore, in their end-use application of their pharmacological characteristics, the compounds of this invention (I) are useful in the treatment of Parkinson's Disease. This end-use application may be either alone or, as in the situation with L-deprenyl, in conjunction with L-DOPA. Further, the end-use application may be in conjunction with a peripheral inhibitor of L-DOPA decarboxylase (e.g., carbidopa).

For pharmacological end-use applications, the compounds of formula I are preferentially administered in the form of their pharmaceutically acceptable acid addition salts. Of course, the effective dosage of the compounds will vary according to the individual potency of each compound employed, the severity and nature of the disease being treated and the particular subject being treated. In general, effective results can be achieved by administering a compound at a dosage of about 0.05 mg to about 10 mg per kilogram of body weight per day, administered systemically. Therapy should be initiated at lower dosages. The dosage thereafter may be administered orally in solid dosage forms, e.g., capsules, tablets, or powders, or in liquid forms, e.g., solutions or suspensions. The compounds may also be injected parenterally in the form of sterile solutions or suspensions. Solid oral forms may contain conventional excipients, for instance: lactose, sucrose, magnesium stearate, resins, and like materials. Liquid oral forms may contain various flavoring, coloring, preserving, stabilizing, buffering, solubilizing, or suspending agents. If desired, additives, such as saline or glucose may be added to make the solutions isotonic.

As is true for most classes of compounds suitable for use as therapeutic agents, certain members are preferred over others. In the instant invention preferred compounds of formula I wherein R₃ represents hydrogen are those wherein R₁ and R₂ are hydrogen, x is zero and y is zero or one, R₄ and R₆ are preferably methyl or ethyl and R₅ is (CH₂)ₘR'₅ with m preferably being one, R'₅ is phenyl or pyridyl or X,Y-substituted phenyl with X and Y preferably being fluoro. In those instances wherein R₃ is other than H, preferred compounds are those wherein R₁ and R₂ are hydrogen, x is zero and y is one, R₄, R₅ and R₆ are preferably lower alkyl with methyl and ethyl being preferred, with R₃ preferably being (CH₂)ₙR'₃ wherein n is zero or one, and R'₃ being as R'₅ is defined when R₃ is H. Specifically preferred compounds are:
benzyldimethylsilylmethanamine,
dimethyl-4-fluorobenzylsilylmethanamine,
ethyl-4-fluorobenzylmethylsilylmethanamine,
β-(dimethyl-4-fluorobenzylsilyl)ethanamine,
(2,4-difluorobenzyl)ethylmethylsilylmethanamine,
(2,4-difluorobenzyl)dimethylsilylmethanamine,
diethyl-4-fluorobenzylsilylmethanamine,
β-(trimethylsilyl)benzeneethanamine,
β-(trimethylsilyl)benzenepropaneamine,
β-(trimethylsilyl)-(4-fluorobenzene)ethanamine, and
β-(ethyldimethylsilyl)-4-chlorobenzenepropanamine and the above defined (see page 3) mono-, di-, tri-, tetra- and pentafluorobenzyl analogs of dimethylsilylmethanamine and dimethylsilylethanamine.

## Claims (Claims for the following Contracting State(s): AT, BE, CH, DE, FR, GB, IT, LI, LU, NL, SE)

1. A compound of the formula (I) 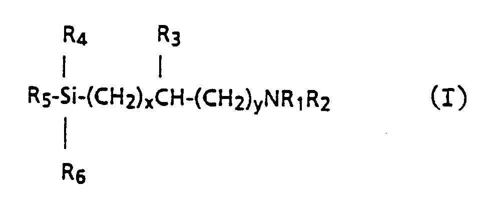 and the pharmaceutically acceptable salts thereof, wherein each of x and y is zero or one, with the proviso that the sum of x and y is less than two,
R₁ is H or C₁₋₁₀ alkyl,
R₂ is H, C₁₋₁₀ alkyl, (CH₂)ₘ phenyl with m being 1 to 4,
R₃ is H or (CH₂)ₙR₃' with n being zero or 1 to 4
R₃' is C₁₋₁₀ alkyl, or aryl;
R₄ is C₁₋₁₀ alkyl,
R₅ is (CH₂)ₘR₅' with m being 1 to 4,
R₆ is (CH₂)ₚR₆' with p being 1 to 4,
with R₃', R₅' and R₆' being H, C₁₋₁₀ alkyl, aryl or X,Y substituted aryl, each of X and Y being C₁₋₁₀ alkyl, C₁₋₁₀ alkoxy, CF₃, halogeno, OH, CN or NO₂, with the proviso that when x and y are zero and R₃ is H then the SiR₄R₅R₆ moiety is other than trimethylsilyl, triethylsilyl, benzyl dimethylsilyl, benzyldiethylsilyl and the further provisos that when x is zero and y is one and SiR₄R₅R₆ is trimethylsilyl then R₃ is other than H, when y is zero and x is one and SiR₄R₅R₆ is trimethylsilyl then R₃ is other than H, methyl or phenyl, and that
Me₃SiCH(Ph)NH₂, Me₃SiCH(o-MePh)NMe₂, Me₃SiCH(Me)NHBz, Et₃Si(CH₂)₃NEt₂, Et₃Si(CH₂)₃N(n-Pr)₂, Et₃Si(CH₂)₃N(n-Bu)₂, Et₃SiCH(Ph)NEt₂, Me₂(m-ClPhCH₂)SiCH₂NHMe, Me₂(m-ClPhCH₂)SiCH₂NHEt, Me₂(m-ClPhCH₂)SiCH₂NHBz, Me₃SiCH(Ph)NMe₂, Me₃SiCH(p-ClPh)NMe₂, Me₃SiCH(p-MePh)NMe₂, Me₃SiCH(p-MeOPh)NMe₂, Me₃SiCH(Me)NHPh, Me₂EtSiCH₂NH₂, Bz₂MeSiCH₂NEt₂, Bz₂MeSiCH₂NMe₂, EtMe₂SiCH₂NH(n-Bu), (n-Pr)Me₂SiCH₂NH(n-Bu), (n-Bu)Me₂SiCH₂NH(n-Bu), MeEt₂SiCH₂NH(n-Bu), Et₃SiCH(Me)CH₂NH₂, Et₃Si(CH₂)₂NEt₂, MeEt₂SiCH₂NH₂, Me₃SiCH(Me)NMe₂, MeEt₂SiCH₂NH₂, Et₂MeSiCH₂NH(n-Bu), (n-Bu)₃Si(CH₂)₂NEt₂, Me₃SiCH(α-naphthyl)NH₂, Me₃SiCH(β-naphthyl)NH₂, Me₃SiCH(Ph)NMe₂, Me₃SiCH(Me)NEt₂, Me₃SiCH(Et)N(n-Pr)₂ are excluded.

2. A compound of claim 1 wherein aryl is phenyl, furyl, thienyl, pyridyl, indolyl or indazolyl.

3. A compound of claim 2 wherein one of R₅ and R₆ is benzyl.

4. A compound of claim 3 wherein the benzyl moiety has at least one fluoro moiety attached thereto.

5. A compound of claim 3 wherein the benzyl moiety has at least two fluoro moieties attached thereto.

6. A compound of claim 3 wherein the benzyl moiety has at least three fluoro moieties attached thereto.

7. A compound of claim 2 wherein one of R₃', R₅' or R₆' represents phenyl, furylmethyl, thienylmethyl, pyridylmethyl, indolylmethyl or indazolylmethyl.

8. A compound of claim 7 wherein m is one and R'₅ is halo substituted phenyl.

9. A compound of claim 8 wherein x and y are zero.

10. A compound of claim 8 wherein one of x and y is one.

11. A compound of claim 1, said compound being dimethyl-4-fluorobenzylsilylmethanamine.

12. A compound of claim 1, said compound being dimethyl(3-pyridylmethyl)silylmethanamine.

13. A compound of claim 1, said compound being β-(dimethyl-4-fluorobenzylsilyl)ethanamine.

14. A compound of claim 1, said compound being 4-chlorobenzyldimethylsilylmethanamine.

15. A compound of claim 1, said compound being (2,4-difluorobenzyl)ethylmethylsilylmethanamine.

16. A compound of claim 1, said compound being (2,4-difluorobenzyl)dimethylsilylmethanamine.

17. A compound of claim 1, said compound being diethyl-4-fluorobenzylsilylmethanamine.

18. A compound of claim 1, said compound being β-(trimethylsilyl)benzeneethanamine.

19. A compound of claim 1, said compound being β-(trimethylsilyl)-(4-fluorobenzene)ethanamine.

20. A process for preparing compounds of the formula (I) according to any one of claims 1 to 19 which comprises reacting a compound of the formula B 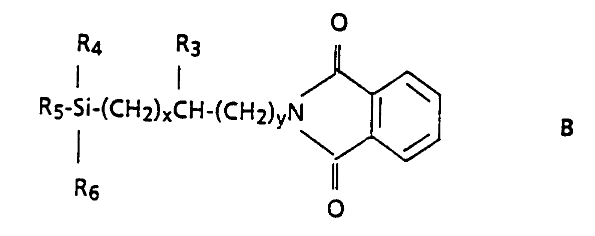 with hydrazine, preferably in its hydrated form, in a suitable solvent, preferably ethanol, and the resulting intermediate treated with an aqueous acid, preferably aqueous hydrochloric acid, said reactions taking place at about the reflux temperature of the reaction mixture, and optionally converting the resulting amines to their R₁R₂-amine derivatives by reaction with the appropriate C₁₋₁₀ alkyl or (CH₂)₁₋₄phenyl halides under standard N-alkylation procedures and/or to their pharmaceutically acceptable salts.

21. A process for the preparation of compounds of the formula (I) according to claim 8 which comprises reacting a compound of the formula D 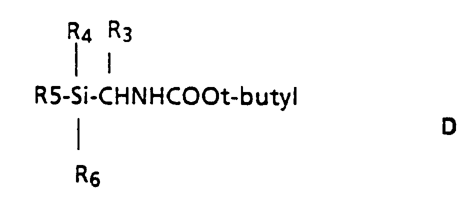 with gaseous hydrochloric acid, in an anhydrous solvent, preferably ether, at about room temperature, and optionally converting the resulting amines to their R₁R₂-amine derivatives by reaction with the appropriate C₁₋₁₀alkyl or (CH₂)₁₋₄phenyl halides under N-alkylation procedures and/or to their pharmaceutically acceptable salts.

22. A process for preparing compounds of the formula (I) according to claim 8 which comprises subjecting a compound of the formula F 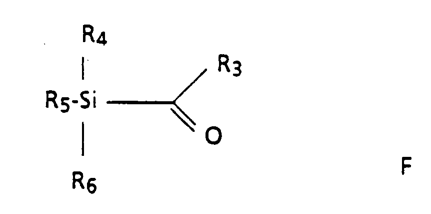 to a reductive amination by reaction of the ketone (F) using sodium cyanoborohydride and ammonium acetate in a suitable anhydrous solvent, preferably methanol at about room temperature, and optionally converting the resulting amines to their R₁R₂-amine derivatives by reaction with the appropriate C₁₋₁₀alkyl or (CH₂)₁₋₄phenyl halides under standard N-alkylation procedures and/or to their pharmaceutically acceptable salts.

23. A pharmaceutical composition containing a compound of the formula (I) 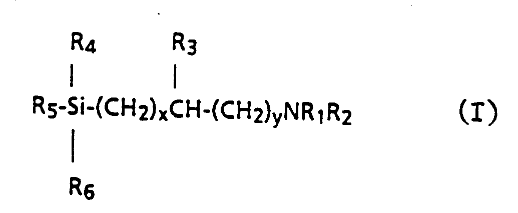 and the pharmaceutically acceptable salts thereof, wherein each of x and y is zero or one, with the proviso that the sum of x and y is less than two,
R₁ is H or C₁₋₁₀ alkyl,
R₂ is H, C₁₋₁₀ alkyl, (CH₂)ₘ phenyl with m being 1 to 4,
R₃ is H or (CH₂)ₙR₃' with n being zero or 1 to 4
R₃' is C₁₋₁₀ alkyl, or aryl;
R₄ is C₁₋₁₀ alkyl,
R₅ is (CH₂)ₘR₅' with m being 1 to 4,
R₆ is (CH₂)ₚR₆' with p being 1 to 4, with R₃', R₅' and R₆' being H, C₁₋₁₀ alkyl, aryl or X,Y substituted aryl, each of X and Y being C₁₋₁₀ alkyl, C₁₋₁₀ alkoxy, CF₃, halogeno, OH, CN or NO₂, with the proviso that when x and y are zero and R₃ is H then the SiR₄R₅R₆ moiety is other than trimethylsilyl, triethylsilyl, benzyl dimethylsilyl, benzyldiethylsilyl and the further provisos that when x is zero and y is one and SiR₄R₅R₆ is trimethylsilyl then R₃ is other than H, when y is zero and x is one and SiR₄R₅R₆ is trimethylsilyl then R₃ is other than H, methyl or phenyl,
together with a pharmaceutically acceptable carrier and/or diluent.

24. The composition according to claim 23 for the treatment of Parkinson's disease.

25. Use of a compound of the formula (I) as defined in claim 23 for the preparation of a pharmaceutical composition.

26. Use according to claim 25 wherein the composition is useful in the treatment of Parkinson's disease.

## Claims (Claims for the following Contracting State(s): ES, GR)

1. A compound of the formula (I) 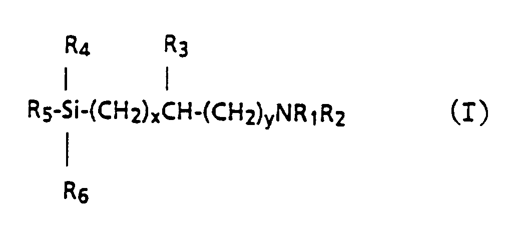 and the pharmaceutically acceptable salts thereof, wherein each of x and y is zero or one, with the proviso that the sum of x and y is less than two,
R₁ is H or C₁₋₁₀ alkyl,
R₂ is H, C₁₋₁₀ alkyl, (CH₂)ₘ phenyl with m being 1 to 4,
R₃ is H or (CH₂)ₙR₃' with n being zero or 1 to 4
R₃' is C₁₋₁₀ alkyl, or aryl;
R₄ is C₁₋₁₀ alkyl,
R₅ is (CH₂)ₘR₅' with m being 1 to 4,
R₆ is (CH₂)ₚR₆' with p being 1 to 4, with R₃', R₅' and R₆' being H, C₁₋₁₀ alkyl, aryl or X,Y substituted aryl, each of X and Y being C₁₋₁₀ alkyl, C₁₋₁₀ alkoxy, CF₃, halogeno, OH, CN or NO₂, with the proviso that when x and y are zero and R₃ is H then the SiR₄R₅R₆ moiety is other than trimethylsilyl, triethylsilyl, benzyl dimethylsilyl, benzyldiethylsilyl and the further provisos that when x is zero and y is one and SiR₄R₅R₆ is trimethylsilyl then R₃ is other than H, when y is zero and x is one and SiR₄R₅R₆ is trimethylsilyl then R₃ is other than H, methyl or phenyl, and that
Me₃SiCH(Ph)NH₂, Me₃SiCH(o-MePh)NMe₂, Me₃SiCH(Me)NHBz, Et₃Si(CH₂)₃NEt₂, Et₃Si(CH₂)₃N(n-Pr)₂, Et₃Si(CH₂)₃N(n-Bu)₂, Et₃SiCH(Ph)NEt₂, Me₂(m-ClPhCH₂)SiCH₂NHMe, Me₂(m-ClPhCH₂)SiCH₂NHEt, Me₂(m-ClPhCH₂)SiCH₂NHBz, Me₃SiCH(Ph)NMe₂, Me₃SiCH(p-ClPh)NMe₂, Me₃SiCH(p-MePh)NMe₂, Me₃SiCH(p-MeOPh)NMe₂, Me₃SiCH(Me)NHPh, Me₂EtSiCH₂NH₂, Bz₂MeSiCH₂NEt₂, Bz₂MeSiCH₂NMe₂, EtMe₂SiCH₂NH(n-Bu), (n-Pr)Me₂SiCH₂NH(n-Bu), (n-Bu)Me₂SiCH₂NH(n-Bu), MeEt₂SiCH₂NH(n-Bu), Et₃SiCH(Me)CH₂NH₂, Et₃Si(CH₂)₂NEt₂, MeEt₂SiCH₂NH₂, Me₃SiCH(Me)NMe₂, MeEt₂SiCH₂NH₂, Et₂MeSiCH₂NH(n-Bu), (n-Bu)₃Si(CH₂)₂NEt₂, Me₃SiCH(α-naphthyl)NH₂, Me₃SiCH(β-naphthyl)NH₂, Me₃SiCH(Ph)NMe₂, Me₃SiCH(Me)NEt₂, Me₃SiCH(Et)N(n-Pr)₂ are excluded.

2. A compound of claim 1 wherein aryl is phenyl, furyl, thienyl, pyridyl, indolyl or indazolyl.

3. A compound of claim 2 wherein one of R₅ and R₆ is benzyl.

4. A compound of claim 3 wherein the benzyl moiety has at least one fluoro moiety attached thereto.

5. A compound of claim 3 wherein the benzyl moiety has at least two fluoro moieties attached thereto.

6. A compound of claim 3 wherein the benzyl moiety has at least three fluoro moieties attached thereto.

7. A compound of claim 2 wherein one of R₃', R₅' or R₆' represents phenyl, furylmethyl, thienylmethyl, pyridylmethyl, indolylmethyl or indazolylmethyl.

8. A compound of claim 7 wherein m is one and R'₅ is halo substituted phenyl.

9. A compound of claim 8 wherein x and y are zero.

10. A compound of claim 8 wherein one of x and y is one.

11. A compound of claim 1, said compound being dimethyl-4-fluorobenzylsilylmethanamine.

12. A compound of claim 1, said compound being dimethyl(3-pyridylmethyl)silylmethanamine.

13. A compound of claim 1, said compound being β-(dimethyl-4-fluorobenzylsilyl)ethanamine.

14. A compound of claim 1, said compound being 4-chlorobenzyldimethylsilylmethanamine.

15. A compound of claim 1, said compound being (2,4-difluorobenzyl)ethylmethylsilylmethanamine.

16. A compound of claim 1, said compound being (2,4-difluorobenzyl)dimethylsilylmethanamine.

17. A compound of claim 1, said compound being diethyl-4-fluorobenzylsilylmethanamine.

18. A compound of claim 1, said compound being β-(trimethylsilyl)benzeneethanamine.

19. A compound of claim 1, said compound being β-(trimethylsilyl)-(4-fluorobenzene)ethanamine.

20. A process for preparing compounds of the formula (I) according to any one of claims 1 to 19 which comprises reacting a compound of the formula B 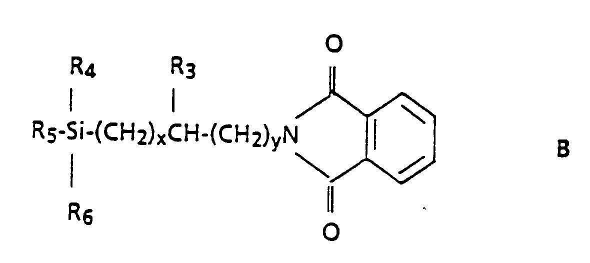 with hydrazine, preferably in its hydrated form, in a suitable solvent, preferably ethanol, and the resulting intermediate treated with an aqueous acid, preferably aqueous hydrochloric acid, said reactions taking place at about the reflux temperature of the reaction mixture, and optionally converting the resulting amines to their R₁R₂-amine derivatives by reaction with the appropriate C₁₋₁₀ alkyl or (CH₂)₁₋₄phenyl halides under standard N-alkylation procedures and/or to their pharmaceutically acceptable salts.

21. A process for the preparation of compounds of the formula (I) according to claim 8 which comprises reacting a compound of the formula D 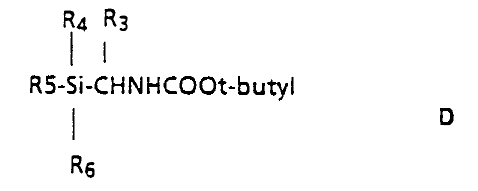 with gaseous hydrochloric acid, in an anhydrous solvent, preferably ether, at about room temperature, and optionally converting the resulting amines to their R₁R₂-amine derivatives by reaction with the appropriate C₁₋₁₀alkyl or (CH₂)₁₋₄phenyl halides under N-alkylation procedures and/or to their pharmaceutically acceptable salts.

22. A process for preparing compounds of the formula (I) according to claim 8 which comprises subjecting a compound of the formula F 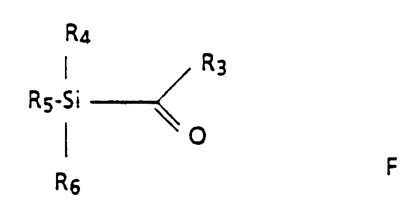 to a reductive amination by reaction of the ketone (F) using sodium cyanoborohydride and ammonium acetate in a suitable anhydrous solvent, preferably methanol at about room temperature, and optionally converting the resulting amines to their R₁R₂-amine derivatives by reaction with the appropriate C₁₋₁₀alkyl or (CH₂)₁₋₄phenyl halides under standard N-alkylation procedures and/or to their pharmaceutically acceptable salts.

23. A method for the preparation of a pharmaceutical composition comprising combining a compound of the formula (I) 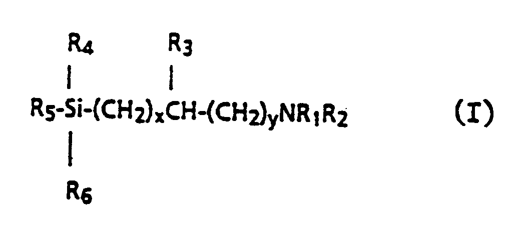 or the pharmaceutically acceptable salts thereof, wherein each of x and y is zero or one, with the proviso that the sum of x and y is less than two,
R₁ is H or C₁₋₁₀ alkyl,
R₂ is H, C₁₋₁₀ alkyl, (CH₂)ₘ phenyl with m being 1 to 4,
R₃ is H or (CH₂)ₙR₃' with n being zero or 1 to 4
R₃' is C₁₋₁₀ alkyl, or aryl;
R₄ is C₁₋₁₀ alkyl,
R₅ is (CH₂)ₘR₅' with m being 1 to 4,
R₆ is (CH₂)ₚR₆' with p being 1 to 4, with R₃', R₅' and R₆' being H, C₁₋₁₀ alkyl, aryl or X,Y substituted aryl, each of X and Y being C₁₋₁₀ alkyl, C₁₋₁₀ alkoxy, CF₃, halogeno, OH, CN or NO₂, with the proviso that when x and y are zero and R₃ is H then the SiR₄R₅R₆ moiety is other than trimethylsilyl, triethylsilyl, benzyl dimethylsilyl, benzyldiethylsilyl and the further provisos that when x is zero and y is one and SiR₄R₅R₆ is trimethylsilyl then R₃ is other than H, when y is zero and x is one and SiR₄R₅R₆ is trimethylsilyl then R₃ is other than H, methyl or phenyl,
with a pharmaceutically acceptable carrier and/or diluent.

24. The method according to claim 23, wherein the pharmaceutical composition prepared is useful in the treatment of Parkinson's disease.

## Patentansprüche (Patentansprüche für folgende(n) Vertragsstaat(en): AT, BE, CH, DE, FR, GB, IT, LI, LU, NL, SE)

1. Verbindung der Formel (I) 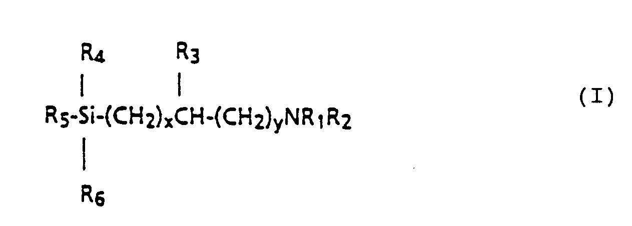 und deren pharmazeutisch verträgliche Salze, in der x und y jeweils 0 oder 1 sind, mit der Maßgabe, daß die Summe von x und y weniger als 2 ist,
R₁ ein Wasserstoffatom oder einen C₁₋₁₀-Alkylrest bedeutet,
R₂ ein Wasserstoffatom, einen C₁₋₁₀-Alkyl- oder (CH₂)ₘ-Phenylrest bedeutet, in dem m 1 bis 4 ist;
R₃ ein Wasserstoffatom oder einen Rest der Formel (CH₂)ₙR₃' bedeutet, in der n 0 oder 1 bis 4 ist,
R₃' einen C₁₋₁₀-Alkyl- oder einen Arylrest bedeutet,
R₄ einen C₁₋₁₀-Alkylrest bedeutet,
R₅ einen Rest der Formel (CH₂)ₘR₅' bedeutet, in der m 1 bis 4 ist,
R₆ einen Rest der Formel (CH₂)ₚR₆' bedeutet, in der p 1 bis 4 ist;
wobei R₃', R₅' und R₆' ein Wasserstoffatom, einen C₁₋₁₀-Alkyl-, einen Aryl- oder X,Y-substituierten Arylrest bedeuten, wobei X und Y jeweils einen C₁₋₁₀-Alkyl-, C₁₋₁₀-Alkoxy-, Trifluormethyl-, Halogen-, Hydroxy-, Cyano- oder Nitrorest bedeuten, mit der Maßgabe, daß, wenn x und y 0 sind und R₃ ein Wasserstoffatom bedeutet, die SiR₄R₅R₆-Einheit verschieden ist von einem Trimethylsilyl-, Triethylsilyl-, Benzyldimethylsilyl- oder Benzyldiethylsilylrest und den weiteren Maßgaben, daß, wenn x den Wert 0 hat, y den Wert 1 hat, und die SiR₄R₅R₆-Einheit ein Trimethylsilylrest ist, R₃ kein Wasserstoffatom ist, daß, wenn y den Wert 0 hat, x den Wert 1 hat und die SiR₄R₅R₆-Einheit ein Trimethylsilylrest ist, R₃ kein Wasserstoffatom, keine Methyl- oder keine Phenylgruppe ist, und daß
Me₃SiCH(Ph)NH₂, Me₃SiCH(o-MePh)NMe₂, Me₃SiCH(Me)NHBz, Et₃Si(CH₂)₃NEt₂, Et₃Si(CH₂)₃N(n-Pr)₂, Et₃Si(CH₂)₃N(n-Bu)₂, Et₃SiCH(Ph)NEt₂, Me₂(m-ClPhCH₂)SiCH₂NHMe, Me₂(m-ClPhCH₂)SiCH₂NHEt, Me₂(m-ClPhCH₂)SiCH₂NHBz, Me₃SiCH(Ph)NMe₂, Me₃SiCH(p-ClPh)NMe₂, Me₃SiCH(p-MePh)NMe₂, Me₃SiCH(p-MeOPh)NMe₂, Me₃SiCH(Me)NHPh, Me₂EtSiCH₂NH₂, Bz₂MeSiCH₂NEt₂, Bz₂MeSiCH₂NMe₂, EtMe₂SiCH₂NH(n-Bu), (n-Pr)Me₂SiCH₂NH(n-Bu), (n-Bu)Me₂SiCH₂NH(n-Bu), MeEt₂SiCH₂NH(n-Bu), Et₃SiCH(Me)CH₂NH₂, Et₃Si(CH₂)₂NEt₂, MeEt₂SiCH₂NH₂, Me₃SiCH(Me)NMe₂, MeEt₂SiCH₂NH₂, Et₂MeSiCH₂NH(n-Bu), (n-Bu)₃Si(CH₂)₂NEt₂, Me₃SiCH(α-naphthyl)NH₂, Me₃SiCH(β-naphthyl)NH₂, Me₃SiCH(Ph)NMe₂, Me₃SiCH(Me)NEt₂, Me₃SiCH(Et)N(n-Pr)₂
ausgenommen sind.

2. Verbindung nach Anspruch 1, in der der Arylrest eine Phenyl-, Furyl-, Thienyl-, Pyridyl-, Indolyl- oder Indazolylgruppe ist.

3. Verbindung nach Anspruch 2, in der einer der Reste R₅ und R₆ eine Benzylgruppe ist.

4. Verbindung nach Anspruch 3, in der an die Benzylgruppe mindestens ein Fluoratom gebunden ist.

5. Verbindung nach Anspruch 3, in der an die Benzylgruppe mindestens zwei Fluoratome gebunden sind.

6. Verbindung nach Anspruch 3, in der an die Benzylgruppe mindestens drei Fluoratome gebunden sind.

7. Verbindung nach Anspruch 2, in der einer der Reste R₃', R₅' oder R₆' eine Phenyl-, Furylmethyl-, Thienylmethyl-, Pyridylmethyl-, Indolylmethyl- oder Indazolylmethylgruppe darstellt.

8. Verbindung nach Anspruch 7, in der m den Wert 1 hat und R₅' ein halogensubstituierter Phenylrest ist.

9. Verbindung nach Anspruch 8, in der x und y den Wert 0 haben.

10. Verbindung nach Anspruch 8, in der eine der Variablen x und y den Wert 1 hat.

11. Verbindung nach Anspruch 1, nämlich Dimethyl-4-fluorbenzylsilylmethanamin.

12. Verbindung nach Anspruch 1, nämlich Dimethyl-(3-pyridylmethyl)silylmethanamin.

13. Verbindung nach Anspruch 1, nämlich β-(Dimethyl-4-fluorbenzylsilyl)ethanamin.

14. Verbindung nach Anspruch 1, nämlich 4-Chlorbenzyldimethylsilylmethanamin.

15. Verbindung nach Anspruch 1, nämlich (2,4-Difluorbenzyl)ethylmethylsilylmethanamin.

16. Verbindung nach Anspruch 1, nämlich (2,4-Difluorbenzyl)dimethylsilylmethanamin.

17. Verbindung nach Anspruch 1, nämlich Diethyl-4-fluorbenzylsilylmethanamin.

18. Verbindung nach Anspruch 1, nämlich β-(Trimethylsilyl)phenylethanamin.

19. Verbindung nach Anspruch 1, nämlich β-(Trimethylsilyl)-(4-fluorphenyl)ethanamin.

20. Verfahren zur Herstellung von Verbindungen der Formel (I) nach einem der Ansprüche 1 bis 19, umfassend die Umsetzung einer Verbindung der Formel B 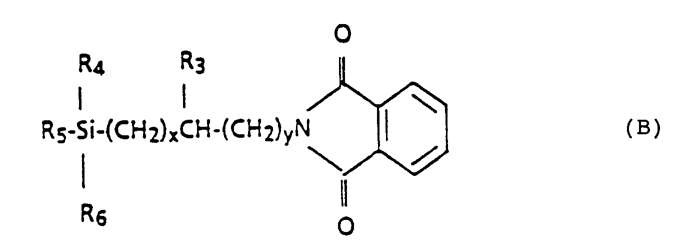 mit Hydrazin, vorzugsweise in Form seines Hydrats, in einem geeigneten Lösungsmittel, vorzugsweise Ethanol, Behandeln des erhaltenen Zwischenprodukts mit einer wäßrigen Säure, vorzugsweise wäßriger Salzsäure, wobei die Reaktionen etwa bei Rückflußtemperatur des Reaktionsgemisches durchgeführt werden, und gegebenenfalls die Umwandlung der erhaltenen Amine in ihre R₁R₂-Aminderivate durch Umsetzung mit geeigneten C₁₋₁₀-Alkyl- oder (CH₂)₁₋₄-Phenylhalogeniden nach üblichen N-Alkylierungsverfahren und/oder in ihre pharmazeutisch verträglichen Salze.

21. Verfahren zur Herstellung von Verbindungen der Formel (I) nach Anspruch 8, umfassend die Umsetzung einer Verbindung der Formel D 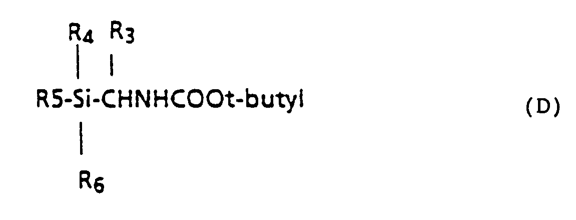 mit gasförmigem Chlorwasserstoff, in einem wasserfreien Lösungsmittel, vorzugsweise Ether, bei etwa Raumtemperatur und gegebenenfalls die Umwandlung der erhaltenen Amine in ihre R₁R₂-Aminderivate durch Umsetzung mit geeigneten C₁₋₁₀-Alkyl- oder (CH₂)₁₋₄-Phenylhalogeniden nach N-Alkylierungsverfahren und/oder in ihre pharmazeutisch verträglichen Salze.

22. Verfahren zur Herstellung von Verbindungen der Formel (I) nach Anspruch 8, umfassend die reduktive Aminierung einer Verbindung der Formel F 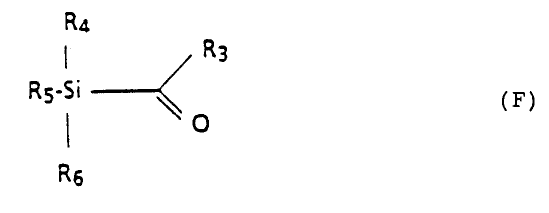 durch Umsetzung des Ketons (F) mit Natriumcyanoborhydrid und Ammoniumacetat in einem geeigneten wasserfreien Lösungsmittel, vorzugsweise Methanol, bei etwa Raumtemperatur und gegebenenfalls Umwandeln der erhaltenen Amine in ihre R₁R₂-Aminderivate durch Umsetzung mit geeigneten C₁₋₁₀-Alkyl- oder (CH₂)₁₋₄-Phenylhalogeniden nach üblichen N-Alkylierungsverfahren und/oder in ihre pharmazeutisch verträglichen Salze.

23. Arzneimittel, enthaltend eine Verbindung der Formel I 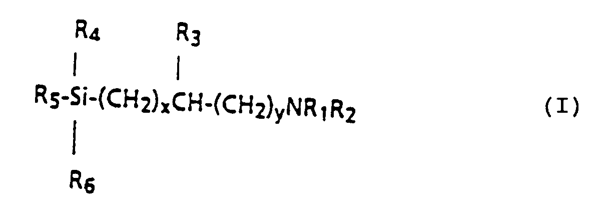 und deren pharmazeutisch verträgliche Salze, wobei x und y jeweils 0 oder 1 sind, mit der Maßgabe, daß die Summe von x und y weniger als 2 ist,
R₁ ein Wasserstoffatom oder einen C₁₋₁₀-Alkylrest bedeutet,
R₂ ein Wasserstoffatom, einen C₁₋₁₀-Alkyl- oder (CH₂)ₘ-Phenylrest bedeutet, in dem m 1 bis 4 ist;
R₃ ein Wasserstoffatom oder einen Rest der Formel (CH₂)ₙR₃' bedeutet, in der n 0 oder 1 bis 4 ist,
R₃' einen C₁₋₁₀-Alkyl- oder einen Arylrest bedeutet,
R₄ einen C₁₋₁₀-Alkylrest bedeutet,
R₅ einen Rest der Formel (CH₂)ₘR₅' bedeutet, in der m 1 bis 4 ist,
R₆ einen Rest der Formel (CH₂)ₚR₆' bedeutet, in der p 1 bis 4 ist,
wobei R₃', R₅' und R₆' ein Wasserstoffatom, einen C₁₋₁₀-Alkyl-, einen Aryl- oder X,Y-substituierten Arylrest bedeuten; wobei X und Y jeweils einen C₁₋₁₀-Alkyl-, C₁₋₁₀-Alkoxy-, Trifluormethyl-, Halogen-, Hydroxy-, Cyano- oder Nitrorest bedeuten, mit der Maßgabe, daß, wenn x und y den Wert 0 haben und R₃ ein Wasserstoffatom bedeutet, die SiR₄R₅R₆-Einheit verschieden ist von einem Trimethylsilyl-, Triethylsilyl-, Benzyldimethylsilyl- oder Benzyldiethylsilylrest; und den weiteren Maßgaben, daß, wenn x den Wert 0 hat, y den Wert 1 hat und die SiR₄R₅R₆-Einheit ein Trimethylsilylrest ist, R₃ kein Wasserstoffatom ist, daß, wenn y den Wert 0 hat, x den Wert 1 hat und die SiR₄R₅R₆-Einheit ein Trimethylsilylrest ist, R₃ kein Wasserstoffatom, keine Methyl- oder keine Phenylgruppe ist, zusammen mit einem pharmazeutisch verträglichen Träger und/oder einem Verdünnungsmittel.

24. Mittel nach Anspruch 23 zur Behandlung der Parkinson'schen Krankheit.

25. Verwendung einer Verbindung der Formel (I), wie in Anspruch 23 definiert zur Herstellung eines Arzneimittels.

26. Verwendung nach Anspruch 25, wobei das Mittel zur Behandlung der Parkinson'schen Krankheit verwendbar ist.

## Patentansprüche (Patentansprüche für folgende(n) Vertragsstaat(en): ES, GR)

1. Verbindung der Formel (I) 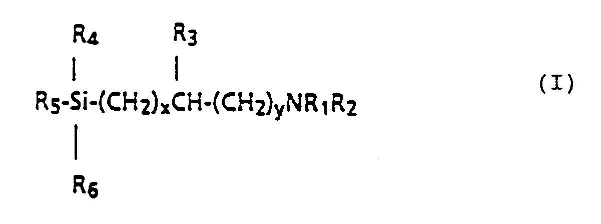 und deren pharmazeutisch verträgliche Salze, in der x und y jeweils 0 oder 1 sind, mit der Maßgabe, daß die Summe von x und y weniger als 2 ist,
R₁ ein Wasserstoffatom oder einen C₁₋₁₀-Alkylrest bedeutet,
R₂ ein Wasserstoffatom, einen C₁₋₁₀-Alkyl- oder (CH₂)ₘ-Phenylrest bedeutet, in dem m 1 bis 4 ist;
R₃ ein Wasserstoffatom oder einen Rest der Formel (CH₂)ₙR₃' bedeutet, in der n 0 oder 1 bis 4 ist,
R₃' einen C₁₋₁₀-Alkyl- oder einen Arylrest bedeutet,
R₄ einen C₁₋₁₀-Alkylrest bedeutet,
R₅ einen Rest der Formel (CH₂)ₘR₅' bedeutet, in der m 1 bis 4 ist,
R₆ einen Rest der Formel (CH₂)ₚR₆' bedeutet, in der p 1 bis 4 ist;
wobei R₃', R₅' und R₆' ein Wasserstoffatom, einen C₁₋₁₀-Alkyl-, einen Aryl- oder X,Y-substituierten Arylrest bedeuten, wobei X und Y jeweils einen C₁₋₁₀-Alkyl-, C₁₋₁₀-Alkoxy-, Trifluormethyl-, Halogen-, Hydroxy-, Cyano- oder Nitrorest bedeuten, mit der Maßgabe, daß, wenn x und y 0 sind und R₃ ein Wasserstoffatom bedeutet, die SiR₄R₅R₆-Einheit verschieden ist von einem Trimethylsilyl-, Triethylsilyl-, Benzyldimethylsilyl- oder Benzyldiethylsilylrest, und den weiteren Maßgaben, daß, wenn x den Wert 0 hat, y den Wert 1 hat und die SiR₄R₅R₆-Einheit ein Trimethylsilylrest ist, R₃ kein Wasserstoffatom ist, daß, wenn y den Wert 0 hat, x den Wert 1 hat und die SiR₄R₅R₆-Einheit ein Trimethylsilylrest ist, R₃ kein Wasserstoffatom, keine Methyl- oder keine Phenylgruppe ist, und daß
Me₃SiCH(Ph)NH₂, Me₃SiCH(o-MePh)NMe₂, Me₃SiCH(Me)NHBz, Et₃Si(CH₂)₃NEt₂, Et₃Si(CH₂)₃N(n-Pr)₂, Et₃Si(CH₂)₃N(n-Bu)₂, Et₃SiCH(Ph)NEt₂, Me₂(m-ClPhCH₂)SiCH₂NHMe, Me₂(m-ClPhCH₂)SiCH₂NHEt, Me₂(m-ClPhCH₂)SiCH₂NHBz, Me₃SiCH(Ph)NMe₂, Me₃SiCH(p-ClPh)NMe₂, Me₃SiCH(p-MePh)NMe₂, Me₃SiCH(p-MeOPh)NMe₂, Me₃SiCH(Me)NHPh, Me₂EtSiCH₂NH₂, Bz₂MeSiCH₂NEt₂, Bz₂MeSiCH₂NMe₂, EtMe₂SiCH₂NH(n-Bu), (n-Pr)Me₂SiCH₂NH(n-Bu), (n-Bu)Me₂SiCH₂NH(n-Bu), MeEt₂SiCH₂NH(n-Bu), Et₃SiCH(Me)CH₂NH₂, Et₃Si(CH₂)₂NEt₂, MeEt₂SiCH₂NH₂, Me₃SiCH(Me)NMe₂, MeEt₂SiCH₂NH₂, Et₂MeSiCH₂NH(n-Bu), (n-Bu)₃Si(CH₂)₂NEt₂, Me₃SiCH(α-naphthyl)NH₂, Me₃SiCH(β-naphthyl)NH₂, Me₃SiCH(Ph)NMe₂, Me₃SiCH(Me)NEt₂, Me₃SiCH(Et)N(n-Pr)₂
ausgenommen sind.

2. Verbindung nach Anspruch 1, in der der Arylrest eine Phenyl-, Furyl-, Thienyl-, Pyridyl-, Indolyl- oder Indazolylgruppe ist.

3. Verbindung nach Anspruch 2, in der einer der Reste R₅ und R₆ eine Benzylgruppe ist.

4. Verbindung nach Anspruch 3, in der an die Benzylgruppe mindestens ein Fluoratom gebunden ist.

5. Verbindung nach Anspruch 3, in der an die Benzylgruppe mindestens zwei Fluoratome gebunden sind.

6. Verbindung nach Anspruch 3, in der an die Benzylgruppe mindestens drei Fluoratome gebunden sind.

7. Verbindung nach Anspruch 2, in der einer der Reste R₃', R₅' oder R₆' eine Phenyl-, Furylmethyl-, Thienylmethyl-, Pyridylmethyl-, Indolylmethyl- oder Indazolylmethylgruppe darstellt.

8. Verbindung nach Anspruch 7, in der m den Wert 1 hat und R₅' ein halogensubstituierter Phenylrest ist.

9. Verbindung nach Anspruch 8, in der x und y den Wert 0 sind.

10. Verbindung nach Anspruch 8, in der eine der Variablen x und y den Wert 1 hat.

11. Verbindung nach Anspruch 1, nämlich Dimethyl-4-fluorbenzylsilylmethanamin.

12. Verbindung nach Anspruch 1, nämlich Dimethyl-(3-pyridylmethyl)silylmethanamin.

13. Verbindung nach Anspruch 1, nämlich β-(Dimethyl-4-fluorbenzylsilyl)ethanamin.

14. Verbindung nach Anspruch 1, nämlich 4-Chlorbenzyldimethylsilylmethanamin.

15. Verbindung nach Anspruch 1, nämlich (2,4-Difluorbenzyl)ethylmethylsilylmethanamin.

16. Verbindung nach Anspruch 1, nämlich (2,4-Difluorbenzyl)dimethylsilylmethanamin.

17. Verbindung nach Anspruch 1, nämlich Diethyl-4-fluorbenzylsilylmethanamin.

18. Verbindung nach Anspruch 1, nämlich β-(Trimethylsilyl)phenylethanamin.

19. Verbindung nach Anspruch 1, nämlich β-(Trimethylsilyl)-(4-fluorphenyl)ethanamin.

20. Verfahren zur Herstellung von Verbindungen der Formel (I) nach einem der Ansprüche 1 bis 19, umfassend die Umsetzung einer Verbindung der Formel B 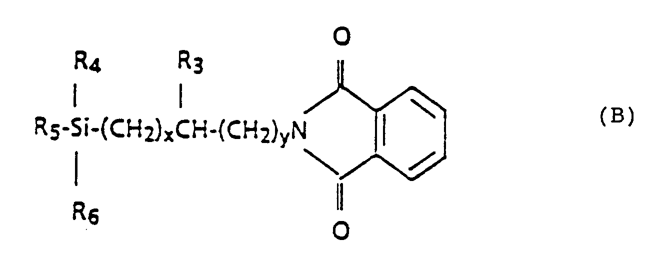 mit Hydrazin, vorzugsweise in Form seines Hydrats, in einem geeigneten Lösungsmittel, vorzugsweise Ethanol, Behandeln des erhaltenen Zwischenprodukts mit einer wäßrigen Säure, vorzugsweise wäßriger Salzsäure, wobei die Reaktionen etwa bei Rückflußtemperatur des Reaktionsgemisches durchgeführt werden, und gegebenenfalls die Umwandlung der erhaltenen Amine in ihre R₁R₂-Aminderivate durch Umsetzung mit geeigneten C₁₋₁₀-Alkyl- oder (CH₂)₁₋₄-Phenylhalogeniden nach üblichen N-Alkylierungsverfahren und/oder in ihre pharmazeutisch verträglichen Salze.

21. Verfahren zur Herstellung von Verbindungen der Formel (I) nach Anspruch 8, umfassend die Umsetzung einer Verbindung der Formel D 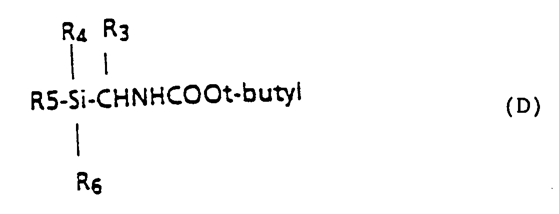 mit gasförmigem Chlorwasserstoff, in einem wasserfreien Lösungsmittel, vorzugsweise Ether, bei etwa Raumtemperatur und gegebenenfalls die Umwandlung der erhaltenen Amine in ihre R₁R₂-Aminderivate durch Umsetzung mit geeigneten C₁₋₁₀-Alkyl- oder (CH₂)₁₋₄-Phenylhalogeniden nach N-Alkylierungsverfahren und/oder in ihre pharmazeutisch verträglichen Salze.

22. Verfahren zur Herstellung von Verbindungen der Formel (I) nach Anspruch 8, umfassend die reduktive Aminierung einer Verbindung der Formel F 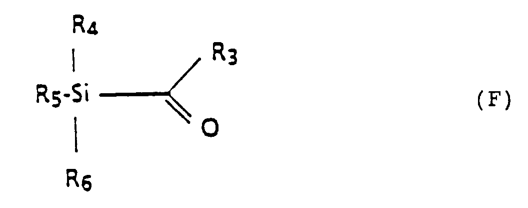 durch Umsetzung des Ketons (F) mit Natriumcyanoborhydrid und Ammoniumacetat in einem geeigneten wasserfreien Lösungsmittel, vorzugsweise Methanol, bei etwa Raumtemperatur und gegebenenfalls Umwandeln der erhaltenen Amine in ihre R₁R₂-Aminderivate durch Umsetzung mit geeigneten C₁₋₁₀-Alkyl- oder (CH₂)₁₋₄-Phenylhalogeniden nach üblichen N-Alkylierungsverfahren und/oder in ihre pharmazeutisch verträglichen Salze.

23. Verfahren zur Herstellung eines Arzneimittels, umfassend das Mischen einer Verbindung der Formel I 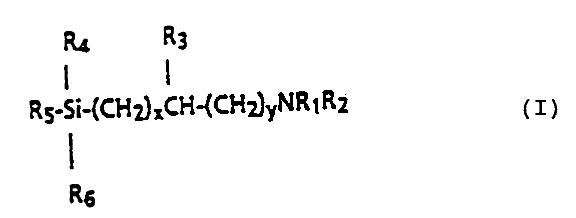 oder deren pharmazeutisch verträglicher Salze, wobei x und y jeweils 0 oder 1 sind, mit der Maßgabe, daß die Summe von x und y weniger als 2 ist,
R₁ ein Wasserstoffatom oder einen C₁₋₁₀-Alkylrest bedeutet,
R₂ ein Wasserstoffatom, einen C₁₋₁₀-Alkyl- oder (CH₂)ₘ-Phenylrest bedeutet, in dem m 1 bis 4 ist;
R₃ ein Wasserstoffatom oder einen Rest der Formel (CH₂)ₙR₃' bedeutet, in der n 0 oder 1 bis 4 ist,
R₃' einen C₁₋₁₀-Alkyl- oder einen Arylrest bedeutet,
R₄ einen C₁₋₁₀-Alkylrest bedeutet,
R₅ einen Rest der Formel (CH₂)ₘR₅' bedeutet, in der m 1 bis 4 ist,
R₆ einen Rest der Formel (CH₂)ₚR₆' bedeutet, in der p 1 bis 4 ist,
wobei R₃', R₅' und R₆' ein Wasserstoffatom, einen C₁₋₁₀-Alkyl-, einen Aryl- oder X,Y-substituierten Arylrest bedeuten; wobei X und Y jeweils einen C₁₋₁₀-Alkyl-, C₁₋₁₀-Alkoxy-, Trifluormethyl-, Halogen-, Hydroxy-, Cyano- oder Nitrorest bedeuten, mit der Maßgabe, daß, wenn x und y den Wert 0 haben und R₃ ein Wasserstoffatom bedeutet, die SiR₄R₅R₆-Einheit verschieden ist von einem Trimethylsilyl-, Triethylsilyl-, Benzyldimethylsilyl- oder Benzyldiethylsilylrest, und den weiteren Maßgaben, daß wenn x den Wert 0 hat, y den Wert 1 hat und die SiR₄R₅R₆-Einheit ein Trimethylsilylrest ist, R₃ kein Wasserstoffatom ist, daß, wenn y den Wert 0 hat, x den Wert 1 hat und die SiR₄R₅R₆-Einheit ein Trimethylsilylrest ist, R₃ kein Wasserstoffatom, keine Methyl- oder keine Phenylgruppe ist,
mit einem pharmazeutisch verträglichen Träger und/oder Verdünnungsmittel.

24. Verfahren nach Anspruch 23, wobei das Arzneimittel zur Behandlung der Parkinson'schen Krankheit verwendbar ist.

## Revendications (Revendications pour l'(les) Etat(s) contractant(s) suivant(s): AT, BE, CH, DE, FR, GB, IT, LI, LU, NL, SE)

1. Un composé de formule (I) : 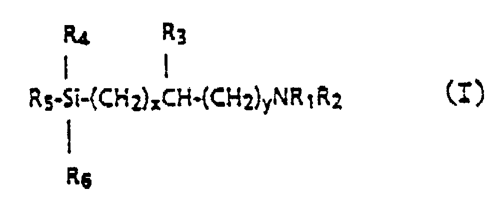 et les sels pharmaceutiquement acceptables dudit composé, formule dans laquelle x et y représentent chacun 0 ou 1, à la condition que la somme de x et de y soit inférieure à 2,
R₁ est H ou un groupe C₁₋₁₀ alkyle,
R₂ est H, ou un groupe C₁₋₁₀ alkyle, (CH₂)ₘ phényle avec m égal à 1 à 4,
R₃ est H ou un groupe (CH₂)ₙR₃' avec n égal à 0 ou 1 à 4,
R₃' est un groupe C₁₋₁₀ alkyle ou aryle ;
R₄ est un groupe C₁₋₁₀ alkyle,
R₅ est un groupe (CH₂)ₘR₅' avec m égal à 1 à 4,
R₆ est un groupe (CH₂)ₚR₆' avec p égal à 1 à 4,
avec R₃', R₅' et R₆' représentant chacun H, un groupe C₁₋₁₀ alkyle, aryle ou aryle à substitution X, Y, X et Y représentant chacun un groupe C₁₋₁₀ alkyle, C₁₋₁₀ alcoxy, CF₃, halogéno, OH, CN ou NO₂, à la condition que lorsque x et y sont égaux à 0 et R₃ est H alors la portion SiR₄R₅R₆ est autre qu'un groupe triméthylsilyle, triéthylsilyle benzyldiméthylsilyle, benzyldiéthylsilyle, et également à la condition que lorsque x est égal à 0 et y est égal à 1 et SiR₄R₅R₆ est un groupe triméthylsilyle alors R₃ est autre que H, et que lorsque est égal à 0 et x est égal à 1, et SiR₄R₅R₆ est un groupe triméthylsilyle alors R₃ est autre que H, un groupe méthyle ou phényle et que Me₃SiCH(Ph)NH₂, Me₃SiCH(o-MePh)NMe₂, Me₃SiCH(Me)NHBz, Et₃Si(CH₂)₃NEt₂, Et₃Si(CH₂)₃N(n-Pr)₂, Et₃Si(CH₂)₃N(n-Bu)₂, Et₃SiCH(Ph)NEt₂, Me₂(m-ClPhCH₂)SiCH₂NHMe, Me₂(m-ClPhCH₂)SiCH₂NHEt, Me₂(m-ClPhCH₂)SiCH₂NHBz, Me₃SiCH(Ph)NMe₂, Me₃SiCH(p-ClPh)NMe₂. Me₃SiCH(p-MePh)NMe₂, Me₃SiCH(p-MeOPh)NMe₂, Me₃SiCH(Me)NHPh, Me₂EtSiCH₂NH₂, Bz₂MeSiCH₂NEt₂, Bz₂MeSiCH₂NMe₂, EtMe₂SiCH₂NH(n-Bu), (n-Pr)Me₂SiCH₂NH(n-Bu), (n-Bu)Me₂SiCH₂NH(n-Bu), MeEt₂SiCH₂NH(n-Bu), Et₃SiCH(Me)CH₂NH₂, Et₃Si(CH₂)₂NEt₂, MeEt₂SiCH₂NH₂, Me₃SiCH(Me)NMe₂, MeEt₂SiCH₂NH₂, Et₂MeSiCH₂NH(n-Bu), (n-Bu)₃Si(CH₂)₂NEt₂, Me₃SiCH(α-naphtyl)NH₂, Me₃SiCH(β-naphtyl)NH₂, Me₃SiCH(Ph)NMe₂, Me₃SiCH(Me)NEt₂, Me₃SiCH(Et)N(n-Pr)₂ soient exclus.

2. Un composé selon la revendication 1, selon laquelle aryle désigne un groupe phényle, furyle, thiényle, pyridyle, indolyle ou indazolyle.

3. Un composé selon la revendication 2, selon laquelle l'un des R₅ et R₆ est un groupe benzyle.

4. Un composé selon la revendication 3, selon laquelle la portion benzyle a au moins une portion fluoro qui y est attachée.

5. Un composé selon la revendication 3, selon laquelle la portion benzyle a au moins deux portions fluoro qui y sont attachées.

6. Un composé selon la revendication 3, selon laquelle la portion benzyle a au moins trois portions fluoro qui y sont attachées.

7. Un composé selon la revendication 2, selon laquelle l'un des R₃', R₅' ou R₆' représente un groupe phényle, furylméthyle, thiénylméthyle, pyridylméthyle, indolylméthyle ou indazolylméthyle.

8. Un composé selon la revendiction 7, selon laquelle m est égal à 1 et R'₅ est un groupe phényle à substitution halo.

9. Un composé selon la revendication 8, selon laquelle x et y sont égaux à 0.

10. Un composé selon la revendication 8, selon laquelle l'un des x et y est égal à 1.

11. Un composé selon la revendication 1, qui est la diméthyl-4-fluorobenzylsilylméthanamine.

12. Un composé selon la revendication 1, qui est la diméthyl(3-pyridylméthyl)silylméthanamine.

13. Un composé selon la revendication 1, qui est la β-(diméthyl-4-fluorobenzylsilyl)éthanamine.

14. Un composé selon la revendication 1, qui est la 4-chlorobenzyldiméthylsilylméthanamine.

15. Un composé selon la revendication 1, qui est la (2,4-difluorobenzyl)éthylméthylsilylméthanamine.

16. Un composé selon la revendication 1, qui est la (2,4-difluorobenzyl)diméthylsilylméthanamine.

17. Un composé selon la revendication 1, qui est la diéthyl-4-fluorobenzylsilylméthanamine.

18. Un composé selon la revendication 1, qui est la β-(triméthylsilyl)benzèneéthanamine.

19. Un composé selon la revendication 1, qui est la β-(triméthylsilyl)-(4-fluorobenzène)éthanamine.

20. Un procédé de préparation des composés de formule (I) selon l'une quelconque des revendications 1 à 19 qui comprend la réaction d'un composé de formule B : 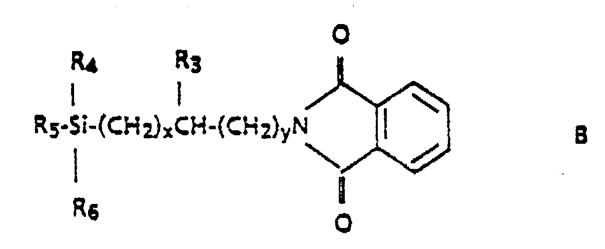 avec l'hydrazine, de préférence sous sa forme hydratée, dans un solvant approprié, de préférence l'éthanol, et l'intermédiaire résultant est traité par un acide aqueux, de préférence l'àcide chlorhydrique aqueux, lesdites réactions prenant place à environ la température de reflux du mélange réactionnel, et la conversion éventuelle des amines résultantes en leurs dérivés R₁R₂-amines par reaction avec les halogénures de C₁₋₁₀ alkyle ou de (CH₂)₁₋₄ phényle appropriés dans les conditions standards de procédé de N-alkylation et/ou en leurs sels pharmaceutiquement acceptables.

21. Un procédé de préparation du composé de formule (I), selon la revendication 8 qui comprend la réaction d'un composé de formule D : 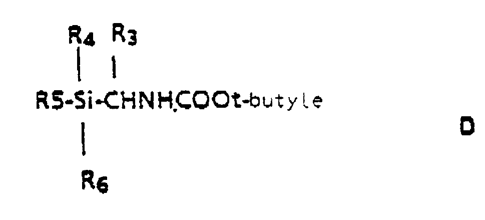 avec l'acide chlorhydrique gazeux, dans un solvant anhydre, de préférence l'éther, à environ la température ambiante et la conversion éventuelle des amines résultantes en leurs dérivés R₁R₂-amines par réaction avec les halogénures de C₁₋₁₀ alkyle ou de (CH₂)₁₋₄ phényle appropriés dans les conditions de procédé de N-alkylation et/ou en leurs sels pharmaceutiquement acceptables.

22. Un procédé de préparation des composés de formule (I), selon la revendication 8 qui consiste à soumettre un composé de formule F : 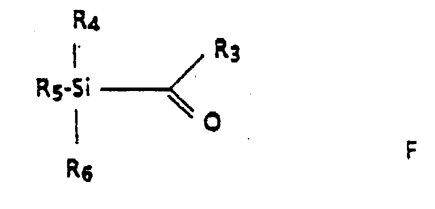 à une amination réductrice par réaction de la cétone (F) utilisant le cyanoborohydrure de sodium et l'acétate d'ammonium dans un solvant anhydre approprié, de préférence le méthanol, à environ la température ambiante, et la conversion éventuelle des amines résultantes en leurs dérivés R₁R₂-amines par réaction avec les halogénures de C₁₋₁₀ alkyle ou de (CH₂)₁₋₄ phényle appropriés dans les conditions standards de procédé de N-alkylation et/ou en leurs sels pharmaceutiquement acceptables.

23. Une composition pharmaceutique contenant un composé de formule (I) : 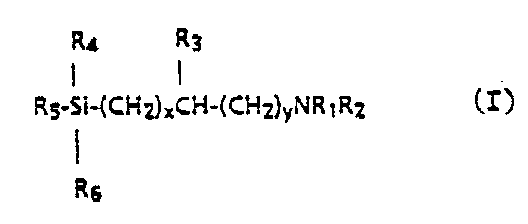 et les sels pharmaceutiquement acceptables dudit composé, formule dans laquelle x et y représentent chacun 0 ou 1, à la condition que la somme de x et de y soit inférieure à 2,
R₁ est H ou un groupe C₁₋₁₀ alkyle,
R₂ est H ou un groupe C₁₋₁₀ alkyle, (CH₂)ₘ phényle avec m égal à 1 à 4,
R₃ est H ou un groupe (CH₂)ₙR₃' avec n égal à 0 ou 1 à 4,
R₃' est un groupe C₁₋₁₀ alkyle ou aryle ;
R₄ est un groupe C₁₋₁₀ alkyle,
R₅ est un groupe (CH₂)ₘR₅' avec m égal à 1 à 4,
R₆ est un groupe (CH₂)ₚR₆' avec p égal à 1 à 4,
avec R₃', R₅' et R₆' représentant chacun H, un groupe C₁₋₁₀ alkyle, aryle ou bien aryle à substitution X, Y, X et Y représentant chacun un groupe C₁₋₁₀ alkyle, C₁₋₁₀ alcoxy, CF₃, halogéno, OH, CN ou NO₂, à la condition que quand x et y sont égaux à 0 et que R₃ est égal H alors la portion SiR₄R₅R₆ est autre qu'un groupe triméthylsilyle, triéthylsilyle benzyldiméthylsilyle, benzyldiethylsilyle et également à la condition que lorsque x est égal à 0 et y est égal à 1 et que SiR₄R₅R₆ est un groupe triméthylsilyle alors R₃ est autre que H et que lorsque y est égal à 0 et x est égal à 1 et que SiR₄R₅R₆ est un groupe triméthylsilyle alors R₃ est autre que H, un groupe méthyle ou phényle,
ensemble avec un support et/ou diluant pharmaceutiquement acceptable.

24. La composition selon la revendication 23 pour le traitement de la maladie de Parkinson.

25. Utilisation d'un composé de formule (I) tel que défini dans la revendication 23 pour la préparation d'une composition pharmaceutique.

26. Utilisation selon la revendication 25, selon laquelle la composition est utile dans le traitement de la maladie de Parkinson.

## Revendications (Revendications pour l'(les) Etat(s) contractant(s) suivant(s): ES, GR)

1. Un composé de formule (I) : 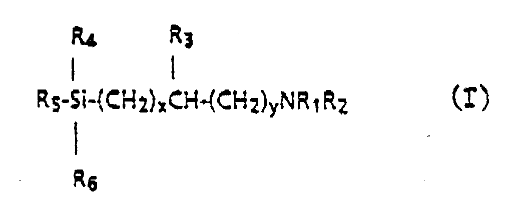 et les sels pharmaceutiquement acceptables dudit composé formule dans laquelle x et y représentent chacun 0 ou 1, à la condition que la somme de x et de y soit inférieure à 2,
R₁ est H ou ùn groupe C₁₋₁₀ alkyle,
R₂ est H, ou un groupe C₁₋₁₀ alkyle, (CH₂)ₘ phényle avec m égal à 1 à 4,
R₃ est H ou un groupe (CH₂)ₙR₃' avec n égal à 0 ou 1 à 4,
R₃' est un groupe C₁₋₁₀ alkyle ou aryle ;
R₄ est un groupe C₁₋₁₀ alkyle,
R₅ est un groupe (CH₂)ₘR₅' avec m égal à 1 à 4,
R₆ est un groupe (CH₂)ₚR₆' avec p égal à 1 à 4,
avec R₃', R₅' et R₆' représentant chacun H, un groupe C₁₋₁₀ alkyle, aryle ou aryle à substitution X, Y, X et Y représentant chacun un groupe C₁₋₁₀ alkyle, C₁₋₁₀ alcoxy, CF₃, halogéno, OH, CN ou NO₂, à la condition que lorsque x et y sont égaux à 0 et R₃ est H, alors la portion SiR₄R₅R₅ est autre qu'un groupe triméthylsilyle, triéthylsilyle, benzyldiméthylsilyle, benzyldiéthylsilyle, et également à la condition que lorsque x est égal à 0 et y est égal à 1 et SiR₄R₅R₆ est un groupe triméthylsilyle, alors R₃ est autre que H, et que lorsque y est égal à 0 et x est égal à 1, et SiR₄R₅R₆ est un groupe triméthylsilyle alors R₃ est autre que H, un groupe méthyle ou phényle, et que Me₃SiCH(Ph)NH₂, Me₃SiCH(o-MePh)NMe₂, Me₃SiCH(Me)NHBz, Et₃Si(CH₂)₃NEt₂, Et₃Si(CH₂)₃N(n-Pr)₂, Et₃Si(CH₂)₃N(n-Bu)₂, Et₃SiCH(Ph)NEt₂, Me₂(m-ClPhCH₂)SiCH₂NHMe, Me₂(m-ClPhCH₂)SiCH₂NHEt, Me₂(m-ClPhCH₂)SiCH₂NHBz, Me₃SiCH(Ph)NMe₂, Me₃SiCH(p-ClPh)NMe₂, Me₃SiCH(p-MePh)NMe₂, Me₃SiCH(p-MeOPh)NMe₂, Me₃SiCH(Me)NHPh, Me₂EtSiCH₂NH₂, Bz₂MeSiCH₂NEt₂, Bz₂MeSiCH₂NMe₂, EtMe₂SiCH₂NH(n-Bu), (n-Pr)Me₂SiCH₂NH(n-Bu), (n-Bu)Me₂SiCH₂NH(n-Bu), MeEt₂SiCH₂NH(n-Bu), Et₃SiCH(Me)CH₂NH₂, Et₃Si(CH₂)₂NEt₂, MeEt₂SiCH₂NH₂, Me₃SiCH(Me)NMe₂, MeEt₂SiCH₂NH₂, Et₂MeSiCH₂NH(n-Bu), (n-Bu)₃Si(CH₂)₂NEt₂, Me₃SiCH(α-naphtyl)NH₂, Me₃SiCH(β-naphtyl)NH₂, Me₃SiCH(Ph)NMe₂, Me₃SiCH(Me)NEt₂, Me₃SiCH(Et)N(n-Pr)₂ soient exclus.

2. Un composé selon la revendication 1, selon laquelle aryle désigne un groupe phényle, furyle, thiényle, pyridyle, indolyle ou indazolyle.

3. Un composé selon la revendication 2, selon laquelle l'un des R₅ et R₆ est un groupe benzyle.

4. Un composé selon la revendication 3, selon laquelle la portion benzyle a au moins une portion fluoro qui y est attachée.

5. Un composé selon la revendication 3, selon laquelle la portion benzyle a au moins deux portion fluoro qui y sont attachées.

6. Un composé selon la revendication 3, selon laquelle la portion benzyle a au moins trois portions fluoro qui y sont attachées.

7. Un composé selon la revendication 2, selon laquelle l'un des R₃', R₅' ou R₆' représente un groupe phényle, furylméthyle, thiénylméthyle, pyridylméthyle, indolylméthyle ou indazolylméthyle.

8. Un composé selon la revendication 7, selon laquelle m est égal à 1 et R'₅ est un groupe phényle à substitution halo.

9. Un composé selon la revendication 8, selon laquelle x et y sont égaux à 0.

10. Un composé selon la revendication 8, selon laquelle l'un des x et y est égal à 1.

11. Un composé selon la revendication 1, qui est la diméthyl-4-fluorobenzysilylméthanamine.

12. Un composé selon la revendication 1, qui est la diméthyl(3-pyridylmethyl)silylméthanamine.

13. Un composé selon la revendication 1, qui est la β-(diméthyl-4-fluorobenzylsilyl)éthanamine.

14. Un composé selon la revendication 1, qui est la 4-chlorobenzyldiméthylsilylméthanamine.

15. Un composé selon la revendication 1, qui est la (2,4-diflurobenzyl)éthylméthylsilylméthanamine.

16. Un composé selon la revendication 1, qui est la (2,4-difluorobenzyl)diméthylsilylméthanamine.

17. Un composé selon la revendication 1, qui est la diéthyl-4-fluorobenzylsilylméthanamine.

18. Un composé selon la revendication 1, qui est la β-(triméthylsilyl)benzèneéthanamine.

19. Un composé selon la revendication 1, qui est la β-(triméthylsilyl)-(4-fluorobenzène)éthanamine.

20. Un procédé de préparation des composés de formule (I) selon l'une quelconque des revendications 1 à 19 qui comprend la réaction d'un composé de formule B : 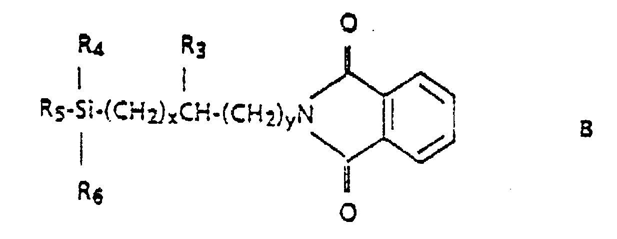 avec l'hydrazine, de préférence sous sa forme hydratée, dans un solvant approprié, de préférence l'éthanol, et l'intermédiaire résultant est traité par un acide aqueux, de préférence l'acide chlorhydrique aqueux, lesdites réactions prenant place à environ la température de reflux du mélange réactionnel, et la conversion éventuelle des amines résultantes en leurs dérivés R₁R₂-amines par réaction avec les halogénures de C₁₋₁₀ alkyle ou de (CH₂)₁₋₄ phényle appropriés dans les conditions standards de procédé de N-alkylation et/ou en leurs sels pharmaceutiquement acceptables.

21. Un procédé de préparation du composé de formule (I), selon la revendication 8 qui comprend la réaction d'un composé de formule D : 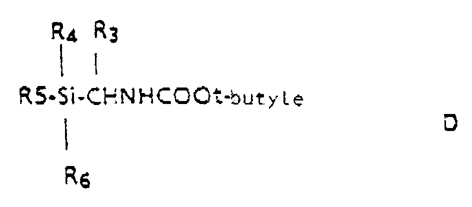 avec l'acide chlorhydrique gazeux, dans un solvant anhydre, de préférence l'éther, à environ la température ambiante, et la conversion éventuelle des amines résultantes en leurs dérivés R₁R₂-amines par réaction avec les halogénures C₁₋₁₀ alkyle ou de (CH₂)₁₋₄ phényle appropriés dans les conditions de procédé de N-alkylation et/ou en leurs sels pharmaceutiquement acceptables.

22. Un procédé de préparation des composés de formule (I), selon la revendication 8 qui consiste à soumettre un composé de formule F : 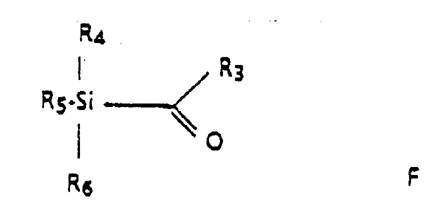 à une amination réductrice par réaction de la cétone (F) utilisant le cyanoborohydrure de sodium et l'acétate d'ammonium dans un solvant anhydre approprié, de préférence le méthanol, à environ la température ambiante, et la conversion éventuelle des amines résultantes en leurs dérivés R₁R₂-amines par réaction avec les halogénures de C₁₋₁₀ alkyle ou de (CH₂)₁₋₄ phényle appropriés dans les conditions standards de procédé de N-alkylation et/ou en leurs sels pharmaceutiquement acceptables.

23. Un procédé pour la préparation d'une composition pharmaceutique comprenant la combinaison d'un composé de formule (I) : 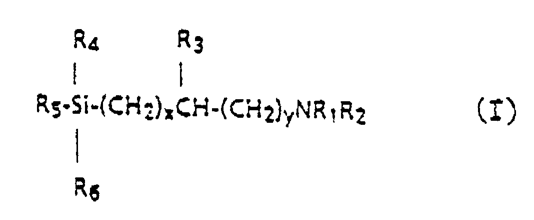 ou les sels pharmaceutiquement acceptables dudit composé, formule dans laquelle x et y représentent chacun 0 ou 1, à la condition que la somme de x et de y soit inférieure à 2,
R₁ est H ou un groupe C₁₋₁₀ alkyle,
R₂ est H ou un groupe C₁₋₁₀ alkyle, (CH₂)ₘ, phényle avec m égal à 1 à 4.
R₃ est H ou un groupe (CH₂)ₙR₃' avec n égal à 0 ou 1 à 4,
R₃' est un groupe C₁₋₁₀ alkyle ou aryle ;
R₄ est un groupe C₁₋₁₀ alkyle,
R₅ est un groupe (CH₂)ₘR₅' avec m égal à 1 à 4,
R₆ est un groupe (CH₂)ₚR₆' avec p égal à 1 à 4,
avec R₃', R₅' et R₆' représentant chacun H, un groupe C₁₋₁₀ alkyle, aryle ou bien aryle à substitution X, Y, X et Y représentant chacun un groupe C₁₋₁₀ alkyle, C₁₋₁₀ alcoxy, CF₃, halogéno, OH, CN ou NO₂, à la condition que quand x et y sont égaux à 0 et que R₃ est égal H alors la portion SiR₄R₅R₆ est autre qu'un groupe triméthylsilyle, triéthylsilyle, benzyldiméthylsilyle, benzyldiéthylsilyle et également à la condition que lorsque x est égal à 0 et y est égal à 1 et que SiR₄R₅R₆ est un groupe triméthylsilyle alors R₃ est autre que H et que lorsque y est égal à 0 et x est égal à 1 et que SiR₄R₅R₆ est un groupe triméthylsilyle alors R₃ est autre que H, un groupe méthyle ou phényle, avec un support et/ou diluant pharmaceutiquement acceptable.

24. Le procédé selon la revendication 23, dans lequel la composition pharmaceutique préparée est pour le traitement de la maladie de Parkinson.
